(19) Europäisches Patentamt
European Patent Office
Office européen des brevets

(11) EP 2 823 816 A1

(12) **EUROPEAN PATENT APPLICATION**

(43) Date of publication:
**14.01.2015 Bulletin 2015/03**

(51) Int Cl.:
***A61K 31/18*** *(2006.01)* ***A61P 35/00*** *(2006.01)*
***A61P 9/10*** *(2006.01)*

(21) Application number: **13175813.8**

(22) Date of filing: **09.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Tragex Pharma**
**92100 Boulogne-Billancourt (FR)**

(72) Inventor: **Hadj-Slimane, Reda**
**75015 Paris (FR)**

(74) Representative: **Icosa**
**83 avenue Denfert-Rochereau**
**75014 Paris (FR)**

(54) **Inhibitors of Neuropilin and use thereof for the treatment of Neuropilin-related diseases**

(57) The present invention relates to a compound of general formula (I),

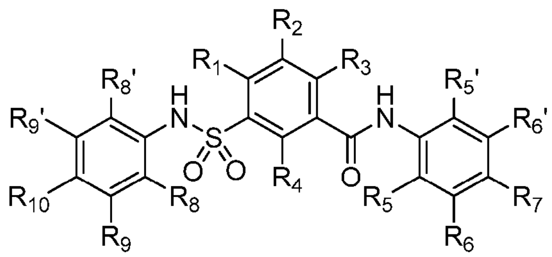

and salts or solvate thereof,
for treating a Neuropilin-related disease, disorder or condition; and to a composition, a pharmaceutical composition and a medicament comprising the compound of general formula (I).

EP 2 823 816 A1

## Description

### FIELD OF INVENTION

The present invention relates to the field of the treatment and/or prevention of diseases, disorders or conditions related to cancers and/or angiogenesis. The present invention particularly relates to specific chemical compounds, herein shown to be inhibitors of the Neuropilin pathways and of VEGF-R receptor, and to the pharmaceutical use thereof, especially for treating and/or preventing diseases, disorders or conditions related to cancers and/or angiogenesis.

### BACKGROUND OF INVENTION

Neuropilin is a transmembrane glycoprotein acting as a co-receptor for two types of ligands: members of the semaphorin family, a family of axon guidance modulators, and members of the vascular endothelial growth factor (VEGF) family. Neuropilin exists in two forms, namely Neuropilin - 1 (NRP-1) and 2 (NRP-2). NRP-1 and NRP-2 present different affinity and specificity of ligands. Both proteins may function as co-receptors binding an extracellular ligand with high affinity, and complexing with other transmembrane receptors of the same ligand (e.g. VEGF-R2 for VEGF), to modulate signal transduction within the cell.

The overexpression of NRP-1 and NRP-2 has been demonstrated to be correlated with tumor progression and patient prognosis in specific tumor types. Moreover, Neuropilin seems to contribute to tumor formation in a number of diseases such as prostate, breast and colon cancer (Ellis LM, Mol Cancer Ther, 2006, 5(5):1099-107). Neuropilin may mediate the effects of VEGF and semaphorins on the proliferation, survival and migration of cancer cells.

Neuropilin may thus be a promising target for preventing and/or treating diseases, disorders or conditions related to cancer.

Moreover, Neuropilin is involved, as a member of the VEGF pathway, in the process of angiogenesis. Angiogenesis is a fundamental process of new blood vessels formation by endothelial cells. This formation involves three different steps, (i) the migration, (ii) the growth and (iii) the differentiation of endothelial cells. Disturbance of this tightly regulated phenomenon may lead to several diseases, wherein tissues and organs are subjected to the invasion by neovessels.

Therefore, an inhibitor of Neuropilin may also be useful for the prevention and/or the treatment of angiogenic diseases, disorders or conditions.

Several means for inhibiting Neuropilin have been described in the art.

For example, WO03/035100 describes a modulator of the expression or activity of Neuropilin.

US7,335,357 describes proteins or protein fragments, especially members of the semaphorin collapsin family, as inhibitors of Neuropilin.

WO2009/099959 and WO99/55855 describe nucleic acids, such as, for example, antisens oligonucleotides, for modulating the expression of Neuropilin genes.

Jarvis et al. (J. Med. Chem. 2010, 53, 2215-26) describes particular chemical compounds (such as, for example, EG00229) for inhibiting the interaction between NRP-1 and VEGF-A.

Moreover, WO2011/143408, in the name of GENENTECH INC., describes antagonists of NRP-1 for use in cancer therapy. WO2011/143408 more specifically describes an anti-NRP-1 antibody, MNRP1685A, which is currently under clinical trials for the treatment of solid tumors.

However, to the Applicant knowledge, there are no advanced-phase clinical trials or current medical development susceptible to lead to a medicament, involving a small molecule inhibiting Neuropilin.

Therefore, due to their great therapeutic potential, there is still a need for a small molecule, such as, for example, a chemical compound, for inhibiting the Neuropilin pathway. Focusing on the identification of a small molecule with NRP-1 inhibitor activity, the Inventors identified chemical compounds of general formula (I),

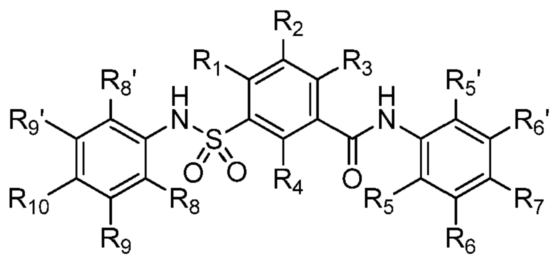

and salts or solvate thereof,

wherein

**$R^1$**, **$R^2$**, **$R^3$** and **$R^4$** are independently a hydrogen atom, or a group selected from alkyl, haloalkyl, alkenyl and haloalkenyl;

**$R^5$** and **$R^{5'}$** are independently a hydrogen atom, or a group selected from alkyl, haloalkyl, alkenyl, haloalkenyl, alkoxy, haloalkoxy, alkylamino and alkylthio, wherein at least one of **$R^5$** and **$R^{5'}$** is an alkoxy, haloalkoxy, alkylamino or alkylthio group;

**$R^6$**, **$R^{6'}$** and **$R^7$** are independently a hydrogen atom, or a group selected from alkyl, haloalkyl, alkenyl, haloalkenyl and alkoxy;

**$R^8$**, **$R^{8'}$**, **$R^9$** and **$R^{9'}$** are independently a hydrogen atom, or a group selected from alkyl, haloalkyl, alkenyl, haloalkenyl and alkoxy;

**$R^{10}$** is a group selected from alkyl, haloalkyl, alkenyl and haloalkenyl.

The present invention also relates to pharmaceutical compositions comprising a compound of general formula (**I**) or salts or solvates thereof, in association with at least one pharmaceutically acceptable vehicle.

The Inventors herein show that these chemical compounds inhibit the interaction between Neuropilin and the components of the VEGF pathway. Without willing to be bound to a theory, the Inventors suggest that the chemical compounds of the invention physically interact with the Neuropilin molecule, and thus block the associated signaling pathways, thereby providing beneficial effect for preventing and/or treating diseases associated to the Neuropilin pathways, such as, for example, cancers (e.g. solid tumor or metastasis) or diseases associated with abnormal angiogenesis (e.g. atherosclerosis, proliferative retinopathies, or rheumatoid arthritis).

## SUMMARY

The present invention thus relates to a compound of general formula (**I**),

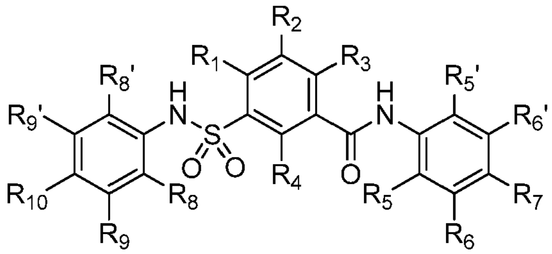

and salts or solvate thereof, wherein **$R^1$, $R^2$, $R^3$, $R^4$, $R^5$, $R^{5'}$, $R^6$, $R^6$, $R^7$, $R^8$, $R^{8'}$**, **$R^9$**, **$R^{9'}$** and **$R^{10}$** are as defined above, for use in the treatment of a Neuropilin-related disease, disorder or condition in a subject in need thereof.

According to one embodiment, the compound for use of the invention is N-(2-ethoxyphenyl)-4-methyl-3-(N-(p-tolyl)sulfamoyl)benzamide or a salt or solvate thereof.

The invention further relates to a composition comprising the compound for use of the invention.

The invention also relates to a pharmaceutical composition comprising a compound for use according to the invention, or salts, solvate or mixtures thereof, in association with at least one pharmaceutically acceptable vehicle.

Another object of the invention is a medicament comprising a compound for use according the invention or salts, solvate or mixtures thereof.

The invention further relates to a method for manufacturing a compound according to the invention, where a compound of general formula (II),

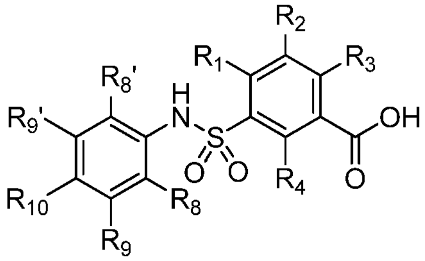

(II)

wherein **$R^1$, $R^2$, $R^3$, $R^4$, $R^8$, $R^{8'}$**, **$R^9$**, **$R^{9'}$**, and **$R^{10}$** are as defined above, is reacted through a peptide coupling reaction

with an amine of general formula (III),

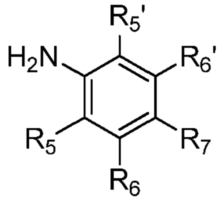

(III)

wherein **$R^5$, $R^{5'}$, $R^6$, $R^{6'}$** and **$R^7$** are as described hereinabove, to give compounds of general formula (I).

According to one embodiment, the invention relates to a compound for use according to the invention; a composition, a pharmaceutical composition or a medicament as defined above; or a compound manufactured by the method described above, wherein the Neuropilin-related disease, disorder or condition is cancer.

According to an embodiment, the invention relates to a compound for use, a composition, a pharmaceutical composition or a medicament for use for inhibiting the proliferation of cancer cells and/or for inhibiting the adhesion of cancer cells and/or for inhibiting the growth of a tumor and/or for inhibiting the number and the development of cancerous cells within the body of the subject and/or for inhibiting the occurrence of metastases.

According to an embodiment, the cancer is one of carcinoma, adenocarcinoma, lymphoma, blastoma, hepatoma, sarcoma; leukemia, such as, for example, squamous cell cancer; lung cancer, including small-cell lung cancer, non-small cell lung cancer, adenocarcinoma of the lung, and squamous carcinoma of the lung; cancer of the peritoneum; hepatocellular cancer; gastric or stomach cancer, including gastrointestinal cancer; pancreatic cancer, such as, for example, pancreatic carcinoma; fibrosarcoma, glioblastoma; neuroblastoma; cervical cancer; ovarian cancer; liver cancer; bladder cancer; hepatoma; breast cancer; colon cancer, such as, for example, colon adenocarcinoma; colorectal cancer, such as, for example, colorectal carcinoma; endometrial or uterine carcinoma; salivary gland carcinoma; osteosarcoma; kidney or renal cancer; liver cancer; prostate cancer, such as, for example, prostate adenocarcinoma; vulval cancer; thyroid cancer; hepatic carcinoma and various types of head and neck cancer; B-cell lymphoma, including low grade/follicular non-Hodgkin's lymphoma (NHL), small lymphocytic (SL) NHL, Burkitt's lymphoma, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, and Waldenstrom's Macroglobulinemia, chronic lymphocytic leukemia (CLL), acute lymphoblastic leukemia (ALL), Hairy cell leukemia, chronic myeloblastic leukemia; and post-transplant lymphoproliferative disorder (PTLD), abnormal vascular proliferation associated with phakomatoses, edema, such as, for example that associated with brain tumors; and Meigs' syndrome.

In an embodiment, the subject is diagnosed with a cancer and/or underwent a surgical operation to remove a tumor and/or was previously treated with an anti-cancer treatment, and/or is simultaneously treated with an anti-cancer treatment, and/or is planned to be treated with an anti-cancer treatment, wherein the anti-cancer treatment may be chemotherapy and/or radiotherapy.

According to one embodiment, the invention relates to a compound for use according to the invention; a composition, a pharmaceutical composition or a medicament as defined above; or a compound manufactured by the method described above, wherein the Neuropilin-related disease, disorder or condition is an angiogenic disease, disorder or condition.

According to one embodiment, the invention relates to a compound for use, a composition, a pharmaceutical composition or a medicament for use for inhibiting the formation of neovessels and/or the adhesion of endothelial cells and/or the growth, proliferation, development or division of endothelial cells.

In one embodiment, the angiogenic disease, disorder or condition is an ophthalmic disease, a disease of the renal and/or urogenital tract; a disease of the digestive tract; a disease of the respiratory tract; a disease of bones or joints; a skin disease; an autoimmune disease; is consecutive to a graft; is a post-surgery reaction, a hyperproliferation or a hypertrophy; abnormal wound healing; chronic inflammation; a disease of the cardiovascular system or of blood or an infection.

## DEFINITIONS

In the present invention, the following terms have the following meanings:

**"Subject"**: refers to an animal, including a human. In the sense of the present invention, a subject may be a patient, i.e. a person receiving medical attention, undergoing or having underwent a medical treatment, or monitored for the

development of a disease.

"**Treating a disease**": refers to both therapeutic treatment and prophylactic or preventative measures; wherein the object is to prevent or slow down (lessen) the targeted neuropilin-related pathologic condition or disorder. Those in need of treatment include those already with the disorder as well as those prone to have the disorder or those in whom the disorder is to be prevented. A subject is successfully "treated" for a neuropilin-related disease or condition if, after receiving a therapeutic amount of a compound according to the present invention, the subject shows observable and/or measurable reduction in or absence of one or more of the following: reduction in the number of pathogenic cells; reduction in the percent of total cells that are pathogenic; and/or relief to some extent, one or more of the symptoms associated with the specific disease or condition; reduced morbidity and mortality, and improvement in quality of life issues. The above parameters for assessing successful treatment and improvement in the disease are readily measurable by routine procedures familiar to a physician. In one embodiment, treating a disease thus includes "**Preventing a disease**", wherein this expression refers to preventing or avoiding the occurrence of symptom. In the present invention, the term "prevention" may refer to a secondary prevention, i.e. to the prevention of the re-occurrence of a symptom or a relapse of the disease. It may also refer, when the disease is cancer, to the occurrence of metastases after the treatment and/or the removal of a tumor.

"**Therapeutically effective amount**": means level or amount of an agent that is aimed at, without causing significant negative or adverse side effects to the target, (1) delaying or preventing the onset of a neuropilin-related disease, disorder, or condition; (2) slowing down or stopping the progression, aggravation, or deterioration of one or more symptoms of the neuropilin-related disease, disorder, or condition; (3) bringing about ameliorations of the symptoms of the neuropilin-related disease, disorder, or condition; (4) reducing the severity or incidence of the neuropilin-related disease, disorder, or condition; or (5) curing the neuropilin-related disease, disorder, or condition. A therapeutically effective amount may be administered prior to the onset of the neuropilin-related disease, disorder, or condition, for a prophylactic or preventive action. Alternatively or additionally, the therapeutically effective amount may be administered after initiation of the neuropilin-related disease, disorder, or condition, for a therapeutic action.

"**Alkyl**": means any saturated linear or branched hydrocarbon chain, with 1 to 6 carbon atoms, preferably 1 to 4 carbon atoms, and more preferably methyl, ethyl, propyl, isopropyl, *n*-butyl, *sec*-butyl, isobutyl and *tert*-butyl.

"**Alkenyl**": means any linear or branched hydrocarbon chain having at least one double bond, of 2 to 6 carbon atoms, and preferably 2 to 4 carbon atoms.

"**Alkoxy**" refers to any O-alkyl group.

"**Alkylthio**" refers to any S-alkyl group.

"**Alkylamino**": means any compound derived from ammoniac $NH_3$ by substitution of one or more hydrogen atoms with an alkyl radical, preferably in the invention, the term amine stands for $N\mathbf{R}_2$ or NH**R**, wherein **R** is an alkyl, preferably a $C_{1-4}$ alkyl.

"**Haloalkyl**" refers to any alkyl group substituted by at least one halogen atom, preferably F, Cl or Br. An example of haloalkyl group is $-CF_3$.

"**Haloalkenyl**" refers to any alkenyl group substituted by at least one halogen atom, preferably F, Cl or Br.

"**Haloalkoxy**" refers to any O-haloalkyl group.

"**Salt**": the compounds of the invention may be in the form of a salt, preferably a pharmaceutically acceptable salt. Pharmaceutically acceptable salts include the acid addition and base salts thereof.

**"Solvate"** is used in the present invention to describe a molecular complex comprising the compound of the invention and one or more pharmaceutically acceptable solvent molecule, for example ethanol. The term "hydrate" is employed when said solvent is water.

**"About":** preceding a figure means plus or less 10% of the value of said figure.

**"$IC_{50}$"** represents the concentration of an inhibitory compound that is required for 50% inhibition in vitro. Methods

for measuring $IC_{50}$ are well-known by the skilled artisan. Examples of such methods include, but are not limited to, ELISA and sandwich ELISA.

**"Pharmaceutically acceptable vehicle"** refers to a vehicle (which may also be referred as an excipient) that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by FDA Office of Biologics standards. The selection of a specific vehicle is dependent on the administration route and on the formulation of the composition of the invention. Examples of pharmaceutically acceptable vehicles include, but are not limited to water, saline, Ringer's solution, dextrose solution, and solutions of ethanol, glucose, sucrose, dextran, mannose, mannitol, sorbitol, polyethylene glycol (PEG), phosphate, acetate, gelatin, collagen, Carbopol™, vegetable oils and the like.

**"Angiogenic disease, disorder or condition"** refers to a disease, disorder or condition associated with angiogenesis, preferably associated with invasive and uncontrolled angiogenesis and/or characterized by undesired, inappropriate, aberrant, excessive and/or pathological vascularization.

**"Cancer"** refers to a broad group of diseases characterized by unregulated cell division and cell growth. As used herein, the term "cancer" may relate to a malignant tumor, such as, for example, a solid tumor; a circulating cancer (hematological cancer); metastases and the like.

**"Neuropilin-related disease, disorder or condition"** includes all medical conditions alleviated by treatment with a Neuropilin inhibitor and includes all diseases, disorders or condition that are acknowledged now, or that will be found in the future, to be associated with Neuropilin activity.

## DETAILED DESCRIPTION

### Compounds

The present invention thus relates to chemical compounds of general formula (I),

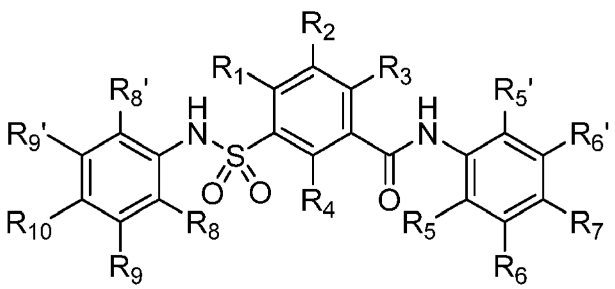

(I)

and salts or solvate thereof,
wherein
**$R^1$, $R^2$, $R^3$** and **$R^4$** are independently a hydrogen atom or a group selected from alkyl, haloalkyl, alkenyl and haloalkenyl; preferably **$R^1$** is a group selected from alkyl, haloalkyl, alkenyl and haloalkenyl and **$R^2$, $R^3$** and **$R^4$** are each a hydrogen atom; more preferably **$R^1$** is an alkyl and **$R^2$**, **$R^3$** and **$R^4$** are each a hydrogen atom;
**$R^5$** and **$R^{5'}$** are independently a hydrogen atom or a group selected from alkyl, haloalkyl, alkenyl, haloalkenyl, alkoxy, haloalkoxy, alkylamino and alkylthio, wherein at least one of **$R^5$** and **$R^{5'}$** is an alkoxy, haloalkoxy, alkylamino or alkylthio group; preferably **$R^5$** is a group selected from alkoxy, haloalkoxy, alkylamino and alkylthio and **$R^{5'}$** is a hydrogen atom or a group selected from alkyl, haloalkyl, alkenyl and haloalkenyl; more preferably **$R^5$** is a group selected from alkoxy, haloalkoxy, alkylamino and alkylthio and **$R^{5'}$** is a hydrogen atom; even more preferably **$R^5$** is an alkoxy group and **$R^{5'}$** is a hydrogen atom;
**$R^6$**, **$R^{6'}$** and **$R^7$** are independently a hydrogen atom, or a group selected from alkyl, haloalkyl, alkenyl, haloalkenyl and alkoxy; preferably **$R^6$**, **$R^{6'}$** and **$R^7$** are independently a hydrogen atom, or a group selected from alkyl and haloalkyl; more preferably **$R^6$**, **$R^{6'}$** and **$R^7$** are each a hydrogen atom;

**$R^8$**, **$R^{8'}$**, **$R^9$** and **$R^{9'}$** are independently a hydrogen atom, or a group selected from alkyl, haloalkyl, alkenyl, haloalkenyl and alkoxy; preferably **$R^8$**, **$R^{8'}$**, **$R^9$** and **$R^{9'}$** are independently a hydrogen atom, or a group selected from alkyl and haloalkyl; more preferably **$R^8$**, **$R^{8'}$**, **$R^9$** and **$R^{9'}$** are each a hydrogen atom;
**$R^{10}$** is a group selected from alkyl, haloalkyl, alkenyl and haloalkenyl; preferably
**$R^{10}$** is a group selected from alkyl and haloalkyl; more preferably **$R^{10}$** is an alkyl group.

In an embodiment, the compound for use of formula (I) of the invention is of general formula (I) wherein
**$R^1$** is a group selected from alkyl, haloalkyl, alkenyl and haloalkenyl;
**$R^2$**, **$R^3$** and **$R^4$** are each a hydrogen atom;
**$R^5$** is a group selected from alkoxy, haloalkoxy, alkylamino and alkylthio;
**$R^{5'}$** is a hydrogen atom or a group selected from alkyl, haloalkyl, alkenyl and haloalkenyl;
**$R^6$**, **$R^{6'}$** and **$R^7$** are independently a hydrogen atom, or a group selected from alkyl and haloalkyl;
**$R^8$**, **$R^{8'}$**, **$R^9$** and **$R^{9'}$** are independently a hydrogen atom, or a group selected from alkyl and haloalkyl;
**$R^{10}$** is a group selected from alkyl and haloalkyl.

In an embodiment, the compound for use of formula (I) of the invention is of general formula (Ia),

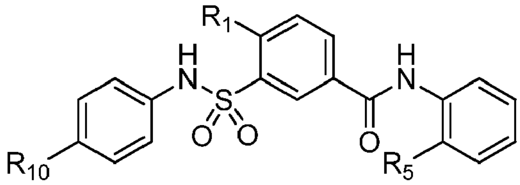

(Ia)

wherein
**$R^1$** is a group selected from alkyl, haloalkyl, alkenyl and haloalkenyl;
**$R^5$** is a group selected from alkoxy, haloalkoxy, alkylamino and alkylthio;
**$R^{10}$** is a group selected from alkyl, haloalkyl, alkenyl and haloalkenyl.

In an embodiment, the compound for use of formula (I) of the invention is of general formula (Ia) wherein
**$R^1$** is an alkyl group; preferably **$R^1$** is an alkyl group selected from methyl, ethyl, propyl, isopropyl, *n*-butyl, *sec*-butyl, isobutyl and *tert*-butyl; more preferably **$R^1$** is a methyl;
**$R^5$** is an alkoxy group; preferably **$R^5$** is an alkoxy group selected from methoxy, ethoxy, propoxy, isopropoxy, *n*-butoxy, *sec*-butoxy, isobutoxy and *tert*-butoxy;
more preferably **$R^5$** is an ethoxy;
**$R^{10}$** is an alkyl group; preferably **$R^{10}$** is an alkyl group selected from methyl, ethyl, propyl, isopropyl, *n*-butyl, *sec*-butyl, isobutyl and *tert*-butyl; more preferably **$R^{10}$** is a methyl.

A preferred compound of formula (I) is:

- N-(2-ethoxyphenyl)-4-methyl-3-(N-(p-tolyl)sulfamoyl)benzamide (compound **1**),

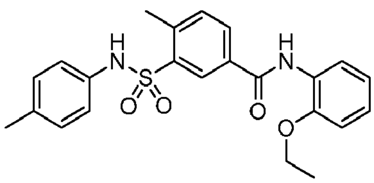

Below are some of the chemical characteristics of the compound **1** of the invention.

| | Compound 1 |
|---|---|
| Chemical name (CAS n°) | 717863-53-1 |
| Molecular formula | $C_{23}H_{24}N_2O_4S_1$ |
| State | Powder |
| Colour | Pink |
| Molecular weight <500 | 424 g/mol |
| H-bond donors <5 | 2 |

(continued)

| | Compound 1 |
|---|---|
| H-bond acceptor <10 | 6 |
| logP <5 | 3.7 |
| Melting point | 176°C |

**Synthesis**

This invention also relates to a method for manufacturing compounds or compositions as hereinabove described, wherein an acid of general formula (II),

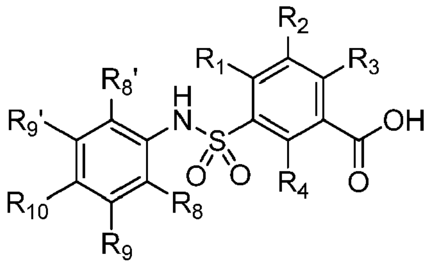

(II)

wherein $\mathbf{R^1}$, $\mathbf{R^2}$, $\mathbf{R^3}$, $\mathbf{R^4}$, $\mathbf{R^8}$, $\mathbf{R^{8'}}$, $\mathbf{R^9}$, $\mathbf{R^{9'}}$ and $\mathbf{R^{10}}$ are as described hereinabove, is reacted through a peptide coupling reaction with an amine of general formula (III),

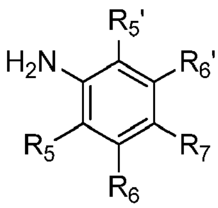

(III)

wherein $\mathbf{R^5}$, $\mathbf{R^{5'}}$, $\mathbf{R^6}$, $\mathbf{R^{6'}}$ and $\mathbf{R^7}$ are as described hereinabove, to give compounds of general formula (I).

In an embodiment, the compound of formula (II) is obtained by reacting a compound of general formula (IV),

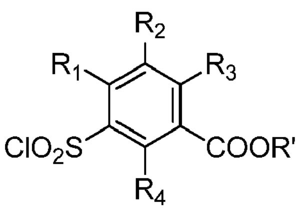

(IV)

wherein $\mathbf{R^1}$, $\mathbf{R^2}$, $\mathbf{R^3}$ and $\mathbf{R^4}$ are as described hereinabove, and **R**' represent an alkyl group, preferably methyl; with a compound of general formula (V),

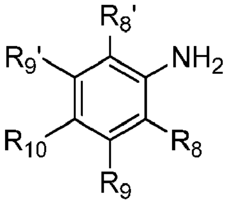

(V)

wherein $\mathbf{R^8}$, $\mathbf{R^{8'}}$, $\mathbf{R^9}$, $\mathbf{R^{9'}}$ and $\mathbf{R^{10}}$ are as described hereinabove, followed by a step of saponification to give compound

of formula (II).

The reaction process is shown below:

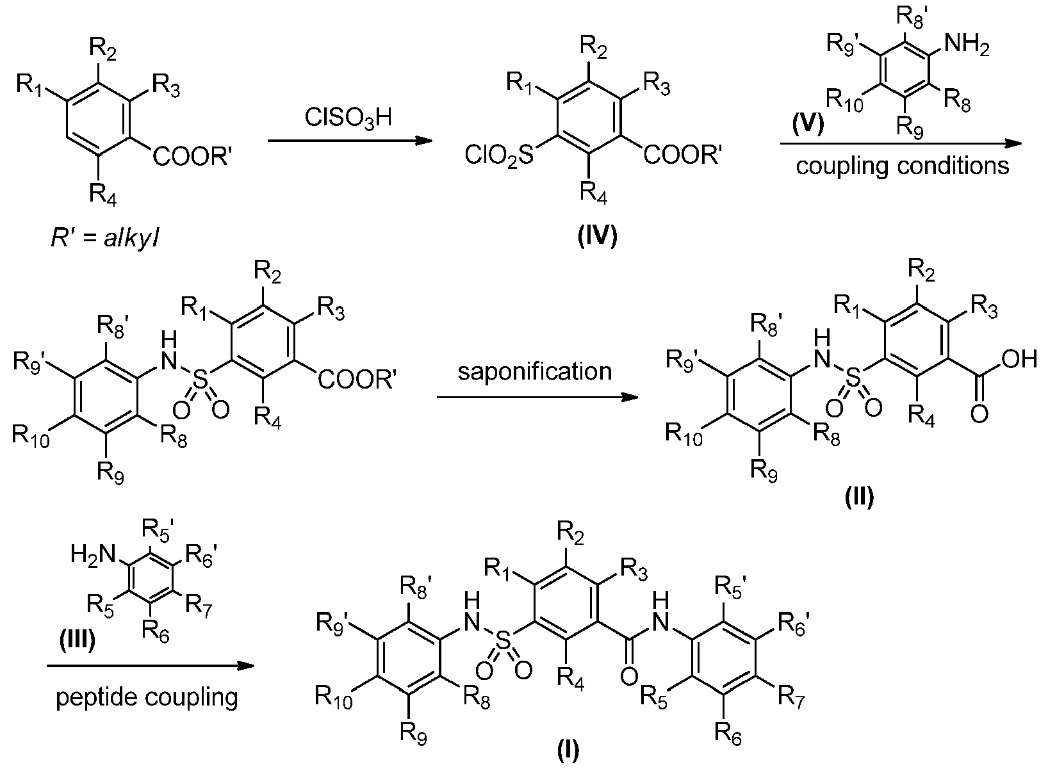


**(IV)** **(II)** **(I)**

The reaction process for synthetizing the compound **1** of the invention is shown below:

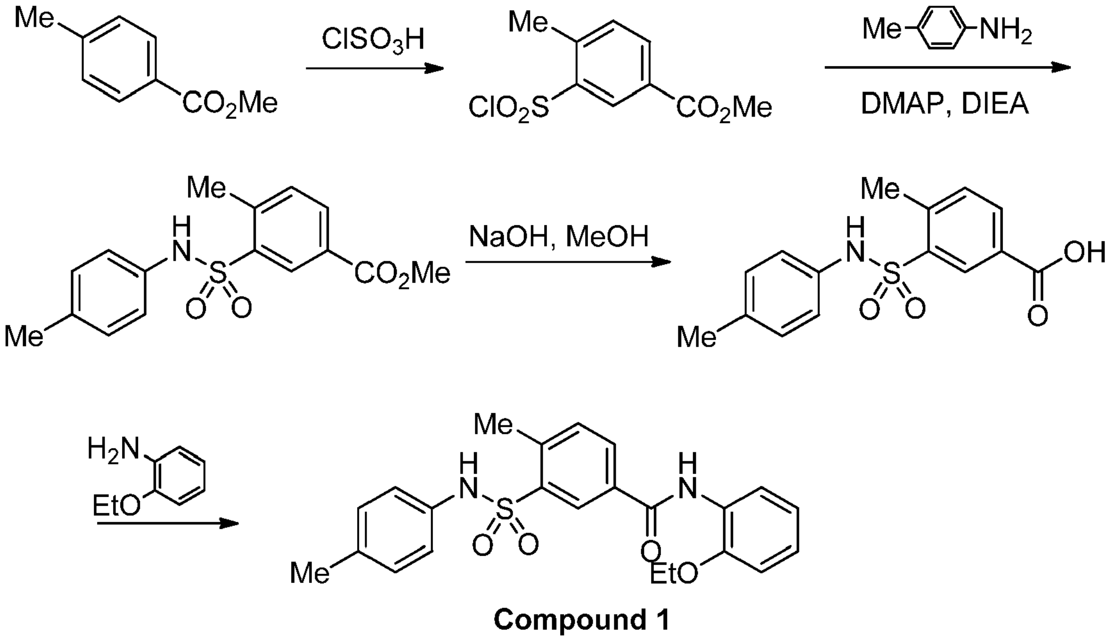


**Compound 1**

## Composition

The present invention also relates to a composition comprising the compound of general formula (I) as hereinabove described, or salts or solvates thereof.

The present invention thus relates to a composition comprising a compound of general formula (I):

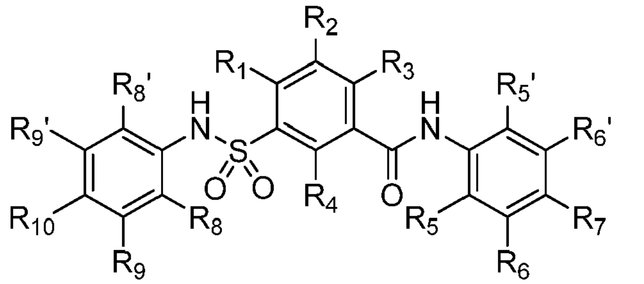

(I)

and salts or solvate thereof,
wherein
**$R^1$**, **$R^2$**, **$R^3$** and **$R^4$** are independently a hydrogen atom or a group selected from alkyl, haloalkyl, alkenyl and haloalkenyl; preferably **$R^1$** is a group selected from alkyl, haloalkyl, alkenyl and haloalkenyl and **$R^2$**, **$R^3$** and **$R^4$** are each a hydrogen atom; more preferably **$R^1$** is an alkyl and **$R^2$**, **$R^3$** and **$R^4$** are each a hydrogen atom;
**$R^5$** and **$R^{5'}$** are independently a hydrogen atom or a group selected from alkyl, haloalkyl, alkenyl, haloalkenyl, alkoxy, haloalkoxy, alkylamino and alkylthio, wherein at least one of **$R^5$** and **$R^{5'}$** is an alkoxy, haloalkoxy, alkylamino or alkylthio group; preferably **$R^5$** is a group selected from alkoxy, haloalkoxy, alkylamino and alkylthio and **$R^{5'}$** is a hydrogen atom or a group selected from alkyl, haloalkyl, alkenyl and haloalkenyl; more preferably **$R^5$** is a group selected from alkoxy, haloalkoxy, alkylamino and alkylthio and **$R^{5'}$** is a hydrogen atom; even more preferably **$R^5$** is an alkoxy group and **$R^{5'}$** is a hydrogen atom;
**$R^6$**, **$R^{6'}$** and **$R^7$** are independently a hydrogen atom, or a group selected from alkyl, haloalkyl, alkenyl, haloalkenyl and alkoxy; preferably **$R^6$**, **$R^{6'}$** and **$R^7$** are independently a hydrogen atom, or a group selected from alkyl and haloalkyl; more preferably **$R^6$**, **$R^{6'}$** and **$R^7$** are each a hydrogen atom;
**$R^8$**, **$R^{8'}$**, **$R^9$** and **$R^{9'}$** are independently a hydrogen atom, or a group selected from alkyl, haloalkyl, alkenyl, haloalkenyl and alkoxy; preferably **$R^8$**, **$R^{8'}$**, **$R^9$** and **$R^{9'}$** are independently a hydrogen atom, or a group selected from alkyl and haloalkyl; more preferably **$R^8$**, **$R^{8'}$**, **$R^9$** and **$R^{9'}$** are each a hydrogen atom;
**$R^{10}$** is a group selected from alkyl, haloalkyl, alkenyl and haloalkenyl; preferably
**$R^{10}$** is a group selected from alkyl and haloalkyl; more preferably **$R^{10}$** is an alkyl group;
in association with at least one pharmaceutically acceptable vehicle.

In one embodiment, the composition of the invention comprises the compound **1** as hereinabove described.

In one embodiment, the composition of the invention comprises several compounds of general formula (I) or salts or solvates thereof. Accordingly, the composition of the invention may comprise a mixture of compounds of general formula (I) or salts or solvates thereof.

**Pharmaceutical composition**

The present invention also relates to a pharmaceutical composition comprising at least one compound of general formula (I) as hereinabove described, or salts or solvates thereof, in combination with at least one pharmaceutically acceptable vehicle. In one embodiment, the pharmaceutical composition of the invention comprises the compound **1** as hereinabove described. In one embodiment, the pharmaceutical composition of the invention comprises the composition as hereinabove described.

**Medicament**

The present invention also relates to a medicament comprising at least one compound of general formula (I) as hereinabove described, or salts or solvates thereof. In one embodiment, the medicament of the invention comprises the compound **1** as hereinabove described. In one embodiment, the medicament of the invention comprises the composition or the pharmaceutical composition as hereinabove described.

**Use of the compounds of the invention**

The inventors surprisingly showed that the compounds of the invention are inhibitors of the Neuropilin pathways. Moreover, as shown in the Examples, the compounds of the invention act on both receptors NRP-1 and NRP-2.

The present invention thus further relates a compound of general formula (I), such as, for example, compound

**1**, or to a composition, pharmaceutical composition or medicament comprising thereof, for, or for use in, inhibiting Neuropilin, and/or Neuropilin-related pathways. In a first embodiment, the compound of general formula (I) is for inhibiting NRP-1 and/or NRP-1 related pathways. In a second embodiment, the compound of general formula (I) is for inhibiting NRP-2 and/or NRP-2 related pathways.

The present invention thus also relates to a method for inhibiting Neuropilin, and/or Neuropilin-related pathways, comprising administering a compound of general formula (I), such as, for example, compound **1**. In a first embodiment, the method is for inhibiting NRP-1 and/or NRP-1 related pathways. In a second embodiment, the method is for inhibiting NRP-2 and/or NRP-2 related pathways.

Moreover, the inventors surprisingly showed that the compounds of the invention inhibited the VEGF - NRP-1 binding (see Examples). Without willing to be bound to a theory, the inventors suggest that the compounds of formula (I) of the invention physically interact with the Neuropilin molecule, and thus block the Neuropilin-associated signaling pathway.

In one embodiment of the invention, the compound of general formula (I) may be used for inhibiting the VEGF - NRP-1 binding. According to one embodiment, the method of the invention is for inhibiting the VEGF - NRP-1 binding. In one embodiment of the invention, the in vitro inhibition of the VEGF - NRP-1 binding by the compound of formula (I) of the invention ranges from about 10 to about 50%, preferably from about 20 to about 40%, more preferably by about 32%; when the compound of formula (I) is used at a concentration ranging from about 1 to about 100 μM, preferably at about 10μ M. Methods for measuring the percentage of binding inhibition in vitro are well-known from the skilled artisan, and include, without limitation, ELISA and sandwich ELISA. An example of such method is carried out in the Examples.

The present invention also relates to a compound as hereinabove described, or a composition, pharmaceutical composition or medicament for, or for use in, treating a Neuropilin-related disease, disorder or condition in a subject in need thereof.

The present invention also relates to a method for treating a Neuropilin-related disease, disorder or condition in a subject in need thereof, comprising administering to the subject a compound, a composition, a pharmaceutical composition or a medicament of the invention.

In one embodiment, a therapeutically effective amount of the compound of formula (I) of the invention is administered to the subject.

## CANCER

In a first embodiment of the invention, the Neuropilin-related disease, disorder or condition is cancer. According to this embodiment, the present invention thus relates to a compound as hereinabove described, or a composition, pharmaceutical composition or medicament for, or for use in, treating cancer in a subject in need thereof.

Indeed, the Inventors herein showed that the compounds of the invention inhibit both the adhesion and proliferation of cancer cells in vitro (Figure 3). Moreover, they demonstrated that the compounds of the invention inhibit the motility of cancer cells in vitro (Figure 4). Motility is an essential step for invasion and metastasis. Finally, they evidenced the efficiency of the compounds of the invention as anti-cancer agents: indeed, the administration of the compounds of the invention to mice injected with tumor cells inhibited the development of tumors (Figure 5). Altogether, these experimental data strongly support the promising anti-cancer potential of the compounds of the invention.

In one embodiment of the invention, the compounds of general formula (I) of the invention, or a composition, pharmaceutical composition or medicament comprising thereof may thus be used for inhibiting the proliferation of cancer cells. Preferably, said inhibition has an $IC_{50}$ less than or equal to about 5 μM, more preferably less than or equal to about 2.5 μM, even more preferably less than or equal to about 1 μM and still even more preferably less than or equal to about 0.8 μM.

In one embodiment of the invention, the compounds of general formula (I) of the invention, or a composition, pharmaceutical composition or medicament comprising thereof may also be used for inhibiting the adhesion of cancer cells. Preferably, said inhibition has an $IC_{50}$ less than or equal to about 5 μM, more preferably less than or equal to about 1 μM, and even more preferably less than or equal to about 0.5 μM.

In one embodiment of the invention, the compounds of general formula (I) of the invention, or a composition, pharmaceutical composition or medicament comprising thereof may be used for inhibiting the number and development of cancer cells within the body of the subject.

In one embodiment of the invention, the compounds of general formula (I) of the invention, or a composition, pharmaceutical composition or medicament comprising thereof may be used for inhibiting the growth of a tumor.

In another embodiment of the invention, the compounds of general formula (I) of the invention, or a composition, pharmaceutical composition or medicament comprising thereof may be used for inhibiting and/or preventing the occurrence of metastases. In one embodiment, the compounds of the invention inhibits the migration speed of cancer cells in vitro by at least about 25%, preferably at least about 50%, more preferably about 75%. Methods for measuring the migration speed of cancer cells in vitro are well-known in the art and include, without limitation, seeding cancer cells on

tissue culture dishes in presence of the tested compound; recording cell movements using a microscope (such as, for example, a computer-controlled Eclipse Ti inverted microscope (Nikon Instruments Europe) equipped with a CCD CoolSNAP HQ2 camera (Roper Scientific, USA)); and analyzing images, such as, for example, using Metamorph7 software (Universal Imaging). An example of method for measuring cancer cell motility is disclosed in the Examples.

According to one embodiment, the present invention thus also relates to a method for treating cancer in a subject in need thereof, comprising administering to the subject a compound, a composition, a pharmaceutical composition or a medicament of the invention.

In one embodiment of the invention, the method is for inhibiting the proliferation of cancer cells. In one embodiment of the invention, the method is for inhibiting the adhesion of cancer cells. In one embodiment of the invention, the method is for inhibiting the number and development of cancer cells within the body of the subject. In one embodiment, the method is for inhibiting the growth of a tumor.

In another embodiment of the invention, the method is for inhibiting and/or preventing the occurrence of metastases.

According to an embodiment, cancer is solid tumor, metastatic cancer, non-metastatic cancer or hematological cancer.

In one embodiment, the cancer may originate in the bladder, blood, bone, bone marrow, brain, breast, cervic area, colon, esophagus, eye and periocular tissues including subconjunctival tissues, duodenum, small intestine, large intestine, rectum, anus, gum, head, kidney, liver, lung, nasopharynx, neck, ovary, pancreas, prostate, skin, stomach, testis, tongue, or uterus.

Examples of cancer include, but are not limited to, fibrosarcoma, carcinoma, adenocarcinoma, lymphoma, blastoma, hepatoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, such as, for example, pancreatic carcinoma, glioblastoma, cervical cancer, ovarian cancer, liver cancer, bladder cancer, hepatoma, breast cancer, colon cancer, such as, for example, colon adenocarcinoma (including a colon adenocarcinoma grade II), colorectal cancer, such as, for example, colorectal carcinoma, endometrial or uterine carcinoma, salivary gland carcinoma, kidney or renal cancer, liver cancer, prostate cancer, such as, for example, prostate adenocarcinoma, vulval cancer, thyroid cancer, osteosarcoma, neuroblastoma, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; Burkitt's lymphoma; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

**Patient**

According to a first embodiment, the subject receiving the compound, the composition, the pharmaceutical composition or the medicament of the invention is diagnosed with a cancer; or is at risk of developing a cancer. Examples of risk factors include, but are not limited to, familial history of cancer, genetic predisposition to cancer, environmental risks such as, for example, exposure to carcinogenic chemicals or other types of carcinogenic agents, diet, clinical factors such as, for example, hormonal deregulation or presence of another cancer-inducing disease, and the like.

According to a second embodiment, the subject underwent a surgical operation to remove a tumor.

According to a third embodiment, the subject was previously treated with an anti-cancer treatment, or is simultaneously treated with an anti-cancer treatment, or is planned to be treated with an anti-cancer treatment. Examples of anti-cancer treatment include, but are not limited to, chemotherapy and radiotherapy.

In the sense of the present invention, these three embodiments are not exclusive: a subject may be concerned by one, two or three of these embodiments.

**ANGIOGENESIS**

In a second embodiment of the invention, the Neuropilin-related disease, disorder or condition is an angiogenic disease, disorder or condition. According to this embodiment, the present invention thus relates to a compound as hereinabove described, or a composition, pharmaceutical composition or medicament comprising thereof, for, or for use in, treating an angiogenic disease, disorder or condition in a subject in need thereof.

Indeed, the Inventors herein showed that the compounds of the invention inhibit both the adhesion and proliferation of endothelial cells in vitro (Figure 1). Moreover, they demonstrated that the compounds of the invention inhibit

the formation of new blood vessels. Finally, they also showed that the compounds of the invention inhibit the motility of endothelial cells, which is an important step for the development of tumors.

In one embodiment of the invention, the compounds of general formula (I) of the invention, or a composition, pharmaceutical composition or medicament comprising thereof may thus be used for inhibiting the proliferation of endothelial cells. Preferably, said inhibition has an $IC_{50}$ less than or equal to about 1 μM, more preferably less than or equal to about 0.5 μM, even more preferably less than or equal to about 0.25 μM and still even more preferably less than or equal to about 0.15 μM.

In one embodiment of the invention, the compounds of general formula (I) of the invention, or a composition, pharmaceutical composition or medicament comprising thereof may also be used for inhibiting the adhesion of endothelial cells. Preferably, said inhibition has an $IC_{50}$ less than or equal to about 1 μM, more preferably less than or equal to about 0.5 μM, even more preferably less than or equal to about 0.25 μM, and still even more preferably less than or equal to about 0.17 μM.

In one embodiment of the invention, the compounds of general formula (I) of the invention, or a composition, pharmaceutical composition or medicament comprising thereof may be used for inhibiting the formation of new blood vessels. In one embodiment, the compounds of the invention inhibit the branch number of neovessels by at least 50%, preferably by at least 75%, more preferably by at least 90%.

In one embodiment of the invention, the compounds of the invention inhibit the migration speed of endothelial cells by at least about 25%, preferably at least about 50%, more preferably about 75%. Methods for measuring the migration speed of endothelial cells in vitro are well-known in the art and include, without limitation, seeding endothelial cells on tissue culture dishes in presence of the tested compound; recording cell movements using a microscope (such as, for example, a computer-controlled Eclipse Ti inverted microscope (Nikon Instruments Europe) equipped with a CCD Cool- SNAP HQ2 camera (Roper Scientific, USA)); and analyzing images, such as, for example, using Metamorph7 software (Universal Imaging). An example of method for measuring endothelial cell motility is disclosed in the Examples.

In one embodiment of the invention, the present invention thus relates to a method for treating an angiogenic disease, disorder or condition in a subject in need thereof, comprising administering to the subject a compound, a composition, a pharmaceutical composition or a medicament of the invention.

In one embodiment of the invention, the method is for inhibiting the proliferation of endothelial cells. In one embodiment of the invention, the method is for inhibiting the adhesion of endothelial cells. In one embodiment of the invention, the method is for inhibiting the formation of new blood vessels.

According to an embodiment, the angiogenic disease, disorder or condition is tumor related. Indeed, the growth and metastasis of tumors are directly linked to angiogenesis: the tumor may stimulate the growth of neovessels, which (i) allow the supply of nutrients and oxygen necessary to its growth, and (2) are escape routes for tumors, facilitating the dissemination of metastatic cells through the blood circulation. Therefore, in one embodiment, the angiogenic disease, disorder or condition is a cancerous or non-cancerous tumor or a metastasis of a tumor. Examples of cancers and of cancerous tumors are listed hereinabove. Examples of non-cancerous tumors include, but are not limited to, angiofibroma and hemangiomas.

According to one embodiment, the angiogenic disease, disorder or condition is an ocular disease, disorder or condition.

In one embodiment, the angiogenic ocular disease, disorder or condition is associated with retinal, peripheral retinal and/or choroidal neovascularization. Examples of such angiogenic diseases include, but are not limited to uveitis, choroiditis, choroidal vasculopathy (including polypoidal choroidal vasculopathy), hypersensitive retinopathy, retinochoroiditis, chorioretinitis, retinal angiomatosis, retinal degeneration, macular degeneration, age related macular degeneration (AMD, including wet and dry AMD), retinal detachment, retinal neovascularisation, proliferative vitreoretinopathy, retinopathy of prematurity (ROP), central serous chorioretinopathy, diabetic retinopathy (including nonproliferative and proliferative diabetic retinopathy), posterior segment trauma, retinal vascular pathologies, retinal telangiectesa, endophthalmitis, macular edema, radiation-induced retinopathy, cystoid macular edema, macular dystrophy (including vitelliform macular dystrophy), diabetic retinopathy, inflammatory pathologies of the retina, ischemic vascular disease, sickle cell anemia, sickle cell retinopathy, sarcoid, syphilis, pseudoxanthoma elasticum, Pagets disease, vein occlusion, artery occlusion, carotid obstructive disease, chronic uveitis/vitritis, fundus flavimaculatus, mycobacterial infections, Lyme's disease, systemic lupus erythematosis, systemic pathologies with implications for the retina, Eales disease, Behcet's disease, infections causing a retinitis or choroiditis, presumed ocular histoplasmosis, Bests disease, myopia, optic pits, Stargarts disease, pars planitis, chronic retinal detachment, hyperviscosity syndromes, toxoplasmosis, toxocariasis, rubella, Vogt-Koyanagi-Harada syndrome, multifocal choroiditis, retinitis pigmentosa, retinischisis, trauma, proliferative diabetic retinopathy, branch retinal vein occlusion, branch retinal arteriolar occlusion, carotid cavernous fistula, sickling hemoglobinopathy (including SS hemoglobinopathy and SC hemoglobinopathy), non-sickling hemoglobinopathy (including AC hemoglobinopathy and AS hemoglobinopathy), IRVAN syndrome (retinal vasculitic disorder characterized by idiopathic retinal vasculitis, an aneurysm, and neuroretinitis), retinal embolization, familial exudative vitreoretinopathy, hyperviscosity syndrome (including leukemia, Waldenstrom macroglobulinemia, multiple myeloma, polycythemia or

myeloproliferative disorder), aortic arch syndrome, Eales disease and post-laser complications.

In one embodiment, the angiogenic ocular disease, disorder or condition is associated with corneal neovascularization. Examples of such angiogenic diseases include, but are not limited to diabetic retinopathy, retinopathy of prematurity, corneal graft rejection, neovascular glaucoma and retrolental fibroplasia, epidemic keratoconjunctivitis, Vitamin A deficiency, contact lens overwear, atopic keratitis, superior limbic keratitis, pterygium keratitis sicca, sjogrens, acne rosacea, phylectenulosis, syphilis, Mycobacteria infections, lipid degeneration, chemical bums, bacterial ulcers, fungal ulcers, Herpes simplex infections, Herpes zoster infections, protozoan infections, Kaposi sarcoma, Mooren ulcer, Terrien's marginal degeneration, marginal keratolysis, trauma, rheumatoid arthritis, systemic lupus, polyarteritis, Wegeners sarcoidosis, Scleritis, Steven's Johnson disease, periphigoid radial keratotomy, and corneal graph rejection.

In one embodiment, the angiogenic ocular disease, disorder or condition is selected from the group comprising diseases associated with rubeosis (neovascularization of the angle) and diseases caused by the abnormal proliferation of fibrovascular or fibrous tissue including all forms of proliferative vitreoretinopathy, whether or not associated with diabetes.

According to another embodiment, the angiogenic disease, disorder or condition is a disease of the renal and/or urogenital tract. Examples of angiogenic diseases of the renal and/or urogenital tract include, but are not limited to, diabetic nephropathy, endometriosis, haemolytic uremic syndrome, hypertensive nephrosclerosis, inflammatory renal disease, such as glomerulonephritis (including mesangioproliferative glomerulonephritis), inhibition of ovulation and corpus luteum formation, inhibition of implantation and inhibition of embryo development in the uterus and nephrotic syndrome.

According to another embodiment, the angiogenic disease, disorder or condition is a disease of the digestive tract. Examples of angiogenic diseases of the digestive tract include, but are not limited to, inflammatory bowel disease.

According to another embodiment, the angiogenic disease, disorder or condition is a disease of the respiratory tract. Examples of angiogenic diseases of the respiratory tract include, but are not limited to, sarcoid, sarcoidosis, Wegeners sarcoidosis, lung inflammation and chronic asthma.

According to another embodiment, the angiogenic disease, disorder or condition is a skin disease. Examples of angiogenic skin diseases include, but are not limited to, psoriasis, acne rosacea, Kaposi's sarcoma, leukemia, Osier-Weber syndrome (Osler-Weber-Rendu), pemphigoid, pseudoxanthoma elasticum, pyogenic granuloma, scleroderma, Steven's Johnson disease and syphilis.

According to another embodiment, the angiogenic disease, disorder or condition is an autoimmune disease. Examples of autoimmune diseases related to angiogenesis include, but are not limited to, chronic asthma, Crohn's disease, pemphigoid, scleroderma, Sogrens syndrome and systemic lupus.

According to another embodiment, the angiogenic disease, disorder or condition is consecutive to a graft. Examples of reactions related to angiogenesis following the graft of an organ or tissue include, but are not limited to, immune rejection of transplanted organs or tissues (including corneal graft rejection), corneal graft neovascularization and diseases occurring after transplants.

According to another embodiment, the angiogenic disease, disorder or condition is a post-surgery reaction, a hyperproliferation or a hypertrophy. Examples of such diseases include, but are not limited to, abnormal proliferation of fibrovascular tissue, hypertrophic scars and hypertrophy following surgery.

According to another embodiment, the angiogenic disease, disorder or condition is a disease of bones or joints. Examples of angiogenic diseases of bones or joints include, but are not limited to, Pagets disease, osteoarthritis, hemophilic joints, psoriatic arthritis, rheumatoid arthritis and nonunion fractures.

According to another embodiment, the angiogenic disease, disorder or condition is abnormal wound healing.

According to another embodiment, the angiogenic disease, disorder or condition is chronic inflammation.

According to another embodiment, the angiogenic disease, disorder or condition is a disease of the cardiovascular system or of blood. Examples of angiogenic diseases of the cardiovascular system or of blood include, but are not limited to, arterial arteriosclerosis, arteriovenous malformations, artery occlusion, atherosclerosis, carotid obstructive disease, diseases and disorders characterized by undesirable vascular permeability, e.g., edema associated with brain tumors, ascites associated with malignancies, hyperviscosity syndromes, pericardial effusion, such as that associated with pericarditis, and pleural effusion, polyarteritis, preeclampsia, vascular adhesions and vein occlusion.

According to another embodiment, the angiogenic disease, disorder or condition is an infection. Examples of infections causing angiogenic disorders include, but are not limited to, bacterial infection, bacterial ulcers, fungal infection, fungal ulcers, Herpes simplex infections, Herpes zoster infections, Lyme's disease, Mycobacteria infections other than leprosy, parasitic diseases, protozoan infections, toxoplasmosis and viral infection.

**Patient**

According to a first embodiment, the subject receiving the compound, the composition, the pharmaceutical composition or the medicament of the invention is diagnosed with an angiogenic disease, disorder or condition; or is at

risk of developing an angiogenic disease, disorder or condition. Examples of risk factors include, but are not limited to, familial history of angiogenic disease, disorder or condition, genetic predisposition to an angiogenic disease, disorder or condition, environmental risks, and the like.

According to a second embodiment, the subject was previously treated with an anti-cancer treatment, or is simultaneously treated with another anti-angiogenic treatment, or is planned to be treated with another anti-angiogenic treatment.

In the sense of the present invention, these two embodiments are not exclusive: a subject may be concerned by one or two of these embodiments.

## ADMINISTRATION ROUTE / FORMULATIONS

According to an embodiment, the compound or the pharmaceutical composition of the invention may be administered by injection, oral administration, topical administration, implant, aerosol, or by nasal, buccal, vaginal, rectal, intratracheal, endoscopic or percutaneous administration.

In one embodiment, the compound, the composition, the pharmaceutical composition or the medicament of the invention is injected, preferably systemically injected. Examples of formulations adapted to systemic injections include, but are not limited to, liquid solutions or suspensions, solid forms suitable for solution in, or suspension in, liquid prior to injection. Examples of systemic injections include, but are not limited to, intravenous, subcutaneous, intramuscular, intradermal and intraperitoneal injection, and perfusion. In another embodiment, the compound, the composition, the pharmaceutical composition or the medicament of the invention is intratumorally or intralesionally injected. Preferably, when injected, the compound, the composition, the pharmaceutical composition or the medicament of the invention is sterile. Methods for obtaining a sterile pharmaceutical composition include, but are not limited to, GMP synthesis (GMP stands for "Good manufacturing practice").

In another embodiment, the compound, the composition, the pharmaceutical composition or the medicament of the invention is orally administered. Examples of formulations adapted to oral administration include, but are not limited to, solid forms, liquid forms and gels. Examples of solid forms adapted to oral administration include, but are not limited to, pill, tablet, capsule, soft gelatin capsule, hard gelatin capsule, caplet, compressed tablet, cachet, wafer, sugar-coated pill, sugar coated tablet, orodispersing/orodisintegrating tablet, powder, solid forms suitable for solution in, or suspension in, liquid prior to oral administration and effervescent tablet. Examples of liquid forms adapted to oral administration include, but are not limited to, solutions, suspensions, drinkable solutions, elixirs, sealed phial, potion, drench, syrup and liquor.

In another embodiment, the compound, the composition, the pharmaceutical composition or the medicament of the invention is topically administered. Topical administration may be particularly advantageous for the treatment of skin diseases, disorders or conditions. Examples of formulations adapted to topical administration include, but are not limited to, patch, such as, for example, transdermic patch, ointment, gel, cream and the like.

In another embodiment, for the treatment of conditions of the lungs or of the respiratory tract, aerosol delivery can be used.

According to an embodiment, the compound, the composition, the pharmaceutical composition or the medicament of the invention is rectally administered, such as, for example, by means of a suppository. The use of rectal administration may be of particular interest for the treatment of diseases, disorders or conditions of the urogenital tract and/or of the digestive tract.

Other examples of administration routes include, but are not limited to, nasal, buccal, vaginal, intratracheal, endoscopic and percutaneous administration.

In one embodiment of the invention, the Neuropilin-related disease, disorder or condition is an ocular disease. According to this embodiment, the compound, the composition, the pharmaceutical composition or the medicament of the invention may be injected, for example intraocularly or periocularly injected. By "intraocularly injected" is meant, in the present invention, injected directly in the interior of the eye, wherein the interior of the eye means any area located within the eyeball, and which generally includes, but is not limited to, any functional (e.g. for vision) or structural tissues found within the eyeball, or tissues or cellular layers that partially or completely line the interior of the eyeball. Specific examples of such areas include the anterior chamber, the posterior chamber, the vitreous cavity, the choroid, the macula, and the retina, and blood vessels and nerves which vascularize or innervate a posterior ocular region or site. By "periocularly injected" is meant, in the present invention, injected in the tissues adjacent to the eyeball, such as, for example, peribulbar, laterobulbar, subconjonctival, sub-tenon or retrobulbar injection. Examples of formulations adapted to intraocular or periocular injection include, but are not limited to, solutions, emulsions or suspensions. According to this embodiment the compound, the composition, the pharmaceutical composition or the medicament of the invention may also be topically applied. Examples of formulations adapted to topical administration to the eye include, but are not limited to, ointments and eye drops. According to this embodiment, the compound, the composition, the pharmaceutical composition or the medicament of the invention may also be an intraocular implant. Examples of intraocular implant

include, but are not limited to, intraocular lenses.

According to an embodiment, the compound, the composition, the pharmaceutical composition or the medicament of the invention is administered preoperatively, and/or postoperatively.

According to an embodiment, the compound, the composition, the pharmaceutical composition or the medicament of the invention is packaged in unitary dosages. Examples of unitary dosages include, but are not limited to, vials, syringe, pill, caplet, capsule, tablet, sealed phial, suppositories, and pouch containing the composition of the invention.

## DOSAGE

In one embodiment, the composition, the medicament or the pharmaceutical composition of the invention comprises from about 0.1 mg to about 50 g of the compound of formula (I) of the invention.

In one embodiment, the concentration the compound of formula (I) in the composition, the medicament or the pharmaceutical composition of the invention ranges from about 0.1 mg to about 50 g per mg or per mL of the composition, pharmaceutical composition or medicament of the invention.

In one embodiment, the composition, the medicament or the pharmaceutical composition of the invention is such that an amount of the compound of formula (I) of the invention ranging from about 1 μg/kg of body weight to about 1 g/kg of body weight is administered to the subject.

The amount of the compounds, composition, pharmaceutical composition or medicament of the invention to be administered to the subject will be decided by the attending physician within the scope of sound medical judgment. Indeed, the specific therapeutically effective amount for any particular subject may depend upon a variety of factors including, for example, the Neuropilin-related disease, disorder or condition to be treated and its severity; as well as the age, body weight, general health, sex and diet of the subject; the route of administration; the duration of the treatment; drugs used in combination or coincidental with the specific polypeptide employed; and the like.

In one embodiment, the therapeutically effective amount ranges from about 0.1 mg to about 50 g of the compound of formula (I) of the invention. In another embodiment, the therapeutically effective amount ranges from about 1 μg/kg of body weight to about 1 g/kg of body weight.

## BRIEF DESCRIPTION OF THE DRAWINGS

**Figure 1** is a combination of graphs showing the *in vitro* effects of compound **1** of the **invention** on endothelial cells (HUVEC cells). (A) Effect of compound **1** of the invention on HUVEC cells adhesion. (B) Effect of compound **1** of the invention on HUVEC cells proliferation. c: concentration of compound **1** of the invention.

**Figure 2** is a combination of graphs showing the in vitro effect of compound **1** of the invention on blood vessels formation. (A) Images showing the effect of compound **1** of the invention on the formation of vessels by HUVEC cells. (B) Effect of compound **1** of the invention on the formation of capillary-like structure. HUVEC cells were seeded on collagen with VEGF-A165-stimulation in presence of compound **1** of the invention. Tube-like structures were visualized 24 hours later and quantified by the number of branching points.

**Figure 3** is a combination of graphs showing the *in vitro* effects of compound **1** of the invention on cancer cells (MDA-MB-231 cells). (A) Effect of compound **1** of the invention on MDA-MB-231 cells adhesion. (B) Effect of compound **1** of the invention on MDA-MB-231cells proliferation. c: concentration of compound **1** of the invention.

**Figure 4** is a graph showing the in vitro effect of compound **1** of the invention on breast cancer cells (MDA-MB-231) migration. The graph shows the difference of migration speed based on the quantification of the motility levels of individual cell by establishing the trajectory of each cell using Metamorph 7 software.

**Figure 5** is a combination of graphs showing the in vivo anti-tumor effect of compound **1** of the invention. (A) Effect of compound **1** of the invention on body weight. (B) Effect of compound **1** of the invention on tumor growth.

## EXAMPLES

The present invention is further illustrated by the following examples.

**Example 1: Synthesis of the compounds of the invention**

***Synthesis of N-(2-ethoxyphenyl)-4-methyl-3-(N-(p-tolyl)sulfamoyl)benzamide (compound 1 of the invention)***

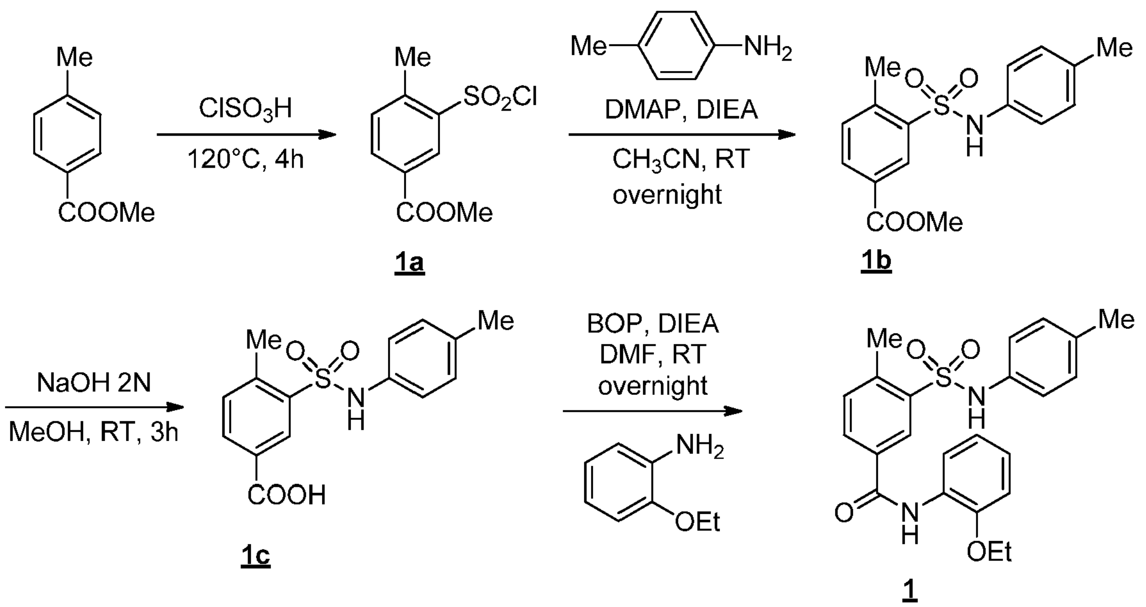


1a 1b 1c 1

The compound 1 was prepared following a four steps sequence. The first step of this synthesis led to the formation of compound **1a** by using methyl *p*-toluate in presence of chlorosulfonic acid. Then, compound **1b** was allowed to react with toluidine and DMAP in acetonitrile provided the compound **1c.** Saponification of compound **1c** was performed by adding NaOH. Finally, compound 1 was obtained by a condensation of compound **1c** and *o*-ethoxyaniline in presence of DMF.

***Synthesis of methyl 3-(chlorosulfonyl)-4-methylbenzoate (1a)***

Chlorosulfonic acid (6 mL, 90.3 mmol) was added dropwise to melt methyl p-toluate (12.7 g, 84.4 mmol) heated at 120°C. The reaction mixture was stirred at this temperature for 4 hours. After completion of the reaction based on TLC monitoring, the reaction mixture was cooled to room temperature, and diluted in water (200 mL). The solution was extracted with ethyl acetate several times, and organic layer was washed with water and brine, dried in $Na_2SO_4$ and concentrated *in vacuo* to give a mixture of two compounds, which are compound **1a** and 3-(chlorosulfonyl)-4-methylbenzoyl chloride.

*$^1H$ NMR ($CDCl_3$)* $\delta$ = 2.9 (s, 3H, $CH_3$); 4.0 (s, 3H, $OCH_3$); 7.55 (d, 1H, H-Ar); 8.3 (d, 1H, H-Ar); 8.7 (s, 1H, H-Ar).

***Synthesis of methyl 4-methyl-3-(N-p-tolylsulfamoyl)benzoate (1b)***

Compound **1a** (4 g, 16.1 mmol) was allowed to react with toluidine (8.5 g, 79.3 mmol) in the presence of catalytic amount of DMAP in acetonitrile (20 mL) at room temperature overnight. Then, water (75 mL) was added and the solution was extracted with ethyl acetate several times. The organic layer was washed with $KHSO_4$ 1M and brine, dried with $Na_2SO_4$ and evaporated to give crude product which will be saponified directly.

*$^1H$ NMR ($CDCl_3$)* $\delta$ = 2.2 (s 3H, $CH_3$); 2.6 (s, 3H, $CH_3$); 3.95 (s, 3H, $OCH_3$); 6.85 (s, 1H, NH); 7.0 (dd, 4H, H-Ar); 7.3 (d, 1H, H-Ar); 8.1 (d, 1H, H-Ar); 8.6 (s, 1H, H-Ar).

***Synthesis of 4-methyl-3-(N-p-tolylsulfamoyl)benzoic acid (1c)***

NaOH 2N (50 mL) was added to a solution of compound **1b** (4.3 g, 20 mmol) in MeOH (50 mL). The mixture was stirred at room temperature for 3 h. MeOH was then evaporated. After that, water (50 mL) was added and a precipitate formed was collected by filtration. The solution was extracted with diethyl oxide several times (50 mL) and the aqueous phase was acidified by HCl 12N. Then, the solution was extracted with ethyl acetate several times and organic layer

was washed with $KHSO_4$ 1M, and brine, dried with $Na_2SO_4$ and evaporated. *p*-toluic acid contained in the residue was eliminated by trituration in ether.

*¹H NMR (DMSO-d6)* $\delta$ = 2.1 (s, 3H, $CH_3$); 2.6 (s, 3H, $CH_3$); 7.0 (dd, 4H, H-Ar); 7.5 (d, 1H, H-Ar); 8.0 (d, 1H, H-Ar); 8.4 (s, 1H, H-Ar); 10.4 (s, 1H, NH); 13 .3 (s, 1H, $CO_2H$).

### *Synthesis of N-(2-ethoxyphenyl)-4-methyl-3-(N-p-tolylsulfamoyl)benzamide (compound 1)*

o-ethoxyaniline (0.7 g, 4.8 mmol), BOP (1.8 g, 4.3 mmol) and DIEA (2 mL, 12.8 mmol) were added to a solution of compound **1c** (1.3 g, 4.0 mmol) in DMF (5 mL). The mixture was stirred at room temperature overnight. Then, ethyl acetate was added and the organic layer was washed with $KHSO_4$ 1M and brine, dried with $Na_2SO_4$ and concentrated *in vacuo.* The crude product was then purified by flash column chromatography on silica gel using ethyl acetate/cyclohexane (1/3) as eluent. The residue obtained was recrystallized in ethanol to obtain compound **1** as pale pink powder (1.8 g).

*¹H NMR (DMSO-d6)* $\delta$ = 1.35 (t, 3H, $CH_3$-$CH_2$); 2.17 (s, 3H, $CH_3$); 2.65 (s, 3H, $CH_3$); 4.1 (q, 2H, $CH_3$-$CH_2$); 6.9-7.1 (m, 7H, H-Ar); 7.5 (d, 1H, H-Ar); 7.7 (d, 1H, H-Ar); 8.05 (d, 1H, H-Ar); 8.2 (s, 1H, H-Ar); 9.06 (s, 1H, NH); 10.38 (s, 1H, NH).

*HPLC* (Vydac C18, 4.6*250mm. A: $H_2O$ with 0.1% TFA, B: 70% acetonitrile with 0.09% TFA. Flow 1mL/min), gradient: 30% B to 100% B in 30 minutes; Rt: 24,6 min.

## Example 2: Characterisation of compound 1 of the invention

| Product | LogP value | MS | Melting point |
|---|---|---|---|
| 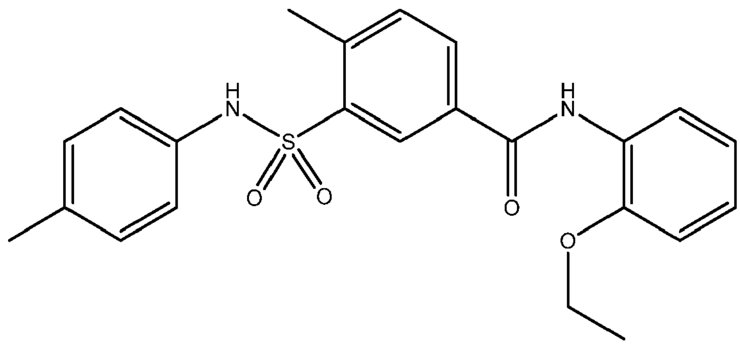 (compound 1) | 3.7 | $MH^+$425 | 176°C |

LogP values were calculated using jlogP program, available at the web address: http://www.vls3d.com/programs.html#section9.

## Example 3: Biological examples

### Methods

#### *Cell culture*

HUVEC (Lonza, Switzerland, cat n° C2517A) were maintained in EBM®-2 (*Lonza, France,* cat n°: CC-3162) supplemented with a kit including 2% foetal bovine serum, human fibroblast growth factor-2 (hFGFB), human epidermal growth factor (hEGF), insulin like growth factor-1 (R3-IGF-1), hydrocortisone, gentamicin and amphotericin-B, heparin and ascorbic acid (Lonza, France, cat n°: CC-4176).

Several cancer cell lines were used: breast cancer cells (MDA-MB-231 (ATCC® Number HTB-26™) and MDA-MB-468 (ATCC® Number HTB-132™ cervical cancer cells (HeLa (ATCC® Number CCL-2™)); colon cancer cells (HCT-116 (ATCC® Number CCL-247™) and HT-29 (ATCC® Number HTB-38™)); pancreas cancer cells (Panc-10.5 (ATCC® Number CRL-2547™)); brain cancer cells (U87 glioblastoma (ATCC® Number HTB-14™), and SH-SYSY neuroblastoma (ATCC® Number CRL-2266™)), lung cancer cells (A549 (ATCC® Number CCL-185™) and NCI-H460 (ATCC® Number HTB-177™)); fibrosarcoma cancer cells (HT-1080 (ATCC® Number CCL-121™)); acute lymphoblastic leukemia cancer

cells (CEM ( ATCC® Number CCL-119™)); Burkitt's lymphoma cancer cells (DAUDI ( ATCC® Number CCL-213™)); B-lymphoma cancer cells (VAL); Mantle cell lymphoma cancer cells (Granta-519) and Chronic myeloid leukemia cancer cells (ERY-1).

The human solid cancer cell lines, including breast cancer cells (MDA-MB-231 and MDA-MB-468); cervical cancer cells (HeLa); colon cancer cells (HCT-116 and HT-29); pancreas cancer cells (Panc-10.5); brain cancer cells (U87 glioblastoma), lung cancer cells (A549 and NCI-H460); and fibrosarcoma cancer cells (HT-1080) were purchased from American Type Culture Collection (ATCC). They were maintained in cultured in DMEM GlutaMAX™ (GIBCO Invitrogen, France, cat n° 31966047) enhanced with 10% foetal bovine serum (GIBCO Invitrogen, France, cat n° 10270-106) and 1% penicillin G/streptomycin (GIBCO Invitrogen, France, cat n° 15140-122). Only Panc-10.5 cells were cultivated in RPMI 1640 medium (Gibco) supplemented with 2 mM L-glutamine, 1.5 g/L sodium bicarbonate, 4.5 g/L glucose, 10 mM HEPES, 1 mM sodium pyruvate, 10 Units/mL human insulin, 85% fetal bovine serum (SVF) : 15% (75 mL in 500 mL of medium).

The human haematological cancer cell lines, including acute lymphoblastic leukemia cancer cells (CEM); Burkitt's lymphoma cancer cells (DAUDI); B-lymphoma cancer cells (VAL); Mantle cell lymphoma cancer cells (Granta-519) and Chronic myeloid leukemia cancer cells (ERY-1) were purchased from ATCC. All cell lines were cultured in RPMI 1640 supplemented with 10% heat-inactivated fetal bovine serum, 2 mmol/L glutamine, and 50 $\mu$g/mL penicillin/streptomycin, at 37°C in a humidified atmosphere containing 5% $CO_2$.

Cells were grown as subconftuent monolayer and cultured in 75cm$^2$ flasks at 37°C, 5% $CO_2$ in a humidified atmosphere, and medium was changed every two days. Only cells from passages 2-6 were used for the experiments.

After observation of cells under the microscope, old media were removed and the adherent cells washed gently twice with PBS (Phosphate Buffer Saline, -$Ca^{2+}$, -$Mg^{2+}$, Lonza cat n°: 17-512F/12). All cells were detached with 3 ml of trypsin/EDTA (chelator of calcium and magnesium ions) (Lonza), incubated for 5 min at 37°C, and examined using a microscope to ensure that the cells were detached and floating. Then, 3 ml TNS (Trypsin Neutralizing Solution, Lonza, France, cat n° CC-5002) or of culture medium were added to inactivate the trypsin, and the cell suspension was transferred in a falcon tube and centrifuged for 7 min at 1400 rpm. The supernatant was removed and the cell pellet resuspended in 10 $\mu$L of culture medium. 50 $\mu$L of this solution were transferred in 20 $\mu$L of isotonic solution and the cells were counted by count cellular (Beckman Coulter). At day 0, 200 $\mu$L of cellular suspension (3.10$^3$ cells/well for the HUVEC cells, breast cancer cells (MDA-MB-231 1.10$^4$ cells/well, MDA-MB-468 2.10$^4$ cells/well,); cervical cancer cells (HeLa 5.000 cells/well); colon cancer cells (HCT-116 5.10$^3$ cells/well, HCT-29 1.1.10$^4$ cells/well); pancreas cancer cells (Panc-10.5 1.5.10$^4$ cells/well); brain cancer cells (U87 5.10$^3$ cells/well, SH-SYSY 8.10$^4$ cells/well); Lung cancer cells (A 549 1.10$^4$ cells/well, NCI-H460 1.10$^4$ cells/well); fibrosarcoma cells (HT 1080 8.10$^3$ cells/well); Burkitt's lymphoma cells (DAUDI 3.10$^4$ cells/well); acute lymphoblastic leukemia cells (CEM 3.10$^4$ cells/well); chronic myeloid leukemia cells (ERY 5.10$^4$ cells/well); mantle cell lymphoma (GRANTA 1.10$^5$ cells/well); B lymphoma cells (VAL 1.10$^5$ cells/well)) were then plated on 96 well plates and incubated at 37°C in a humidified atmosphere of 5% $CO_2$ for 24 h.

At day 1, the compound 1 of the invention was added at increasing concentrations (0.1 - 10 $\mu$M) in the wells and plates were incubated at 37°C in a humidified atmosphere of 5% $CO_2$ for 72 h.

***Adhesion test***

The crystal violet (hexamethylpararosaniline chloride) is used as biologic colorant. It enters in the living cells, allowing the visualisation of the dark purple adherent living cells on the bottom of the plate.

After an incubation period of 72 h, the adherent cells were fixed with 50 mL paraformaldehyde 3% for 15 min at room temperature. The medium and non-adherent cells were removed, and the cells were washed extensively with 100 mL PBS (+$Ca^{2+}$ and +$Mg^{2+}$) (Lonza) and then stained with 50 $\mu$L of crystal violet 0.04% for 30 min at room temperature. The cells were washed gently 3 times with 100 $\mu$L PBS and were permeabilised with 100 $\mu$L triton 2% for 15 min. The microplate was stirred to obtain a homogenous solution and absorbance was quantified at 580 nm by Microplate Manager 5.2.

The percentage of inhibition was calculated as follow: % inhibition = 100- [($DO_{sample}$/ $DO_{control}$) * 100].

***Proliferation assay***

The cell proliferation WST-1 assay is a colorimetric assay for the quantification of cell proliferation and viability, based on the enzymatic cleavage of the tetrazolium salt WST-1 added to the culture medium, to a water soluble colored formazan dye, detected by absorbance at 450 nm, by mitochondrial succinate-tetrazoliumreductase in viable cells. An

augmentation in enzyme activity will then lead to an increase in the amount of formazan dye formed, which directly correlates to the number of metabolically active cells in the culture.

After an incubation period of 72 h, at day 4, were prepared the 19 mL medium with reagent WST-1 1mL (10 mL WST-1 in 200 mL well). The medium was removed from the 96 microplate and 200 μL of solution WST-1/medium were added for each well. The cells were incubated at 37°C in a humidified atmosphere of 5% $CO_2$ for 1h30 and the absorbance at 450-655 nm was measured by Microplate Manager 5.2 (Bio-Rad).

***Motility assay (videomicroscopy)***

Endothelial cells (HUVEC) and breast cancer cells (MDA-MB-231) were seeded in 35 mm diameter tissue culture dishes at $3\times10^4$ and $6\times10^4$ cells per dish respectively and treated separately with DMSO (control) and compound **1** of the invention (treatment) for 24 h, at the concentration of $IC_{50}$ obtained with the proliferation assay.

The dishes were placed in an enclosed warming incubator on the motorized stage of a computer-controlled Eclipse Ti inverted microscope (Nikon Instruments Europe) equipped with a CCD Cool- SNAP HQ2 camera (Roper Scientific, USA) and a PC workstation. The incubator allowed samples to be kept at 37°C in a humidified atmosphere containing 5% $CO_2$, 95% air. Phase images were recorded at 100× every 5 min simultaneously over several fields from 0 till 24 h (288 frames). Cell motility was tracked and analyzed for at least 25 cells in each field over three independent experiments. The total distance of migration and the speed of migration were analyzed. The degree of persistence of movement was defined as the ratio between the linear distance and the total distance covered by the cells. Image acquisition and analyses were carried out using Metamorph7 software (Universal Imaging).

***Tube formation assay***

Collagen type I (1 mg/ml, BD) was mixed with 0.1N NaOH, 0.1% bicarbonate and 10X in medium EBM-2 and kept on ice until placed into the wells. The assay was prepared in 96-well plate format and the bottom layer of collagen was formed by adding 70 μL of the solution per well and allowing subsequent gelation at 37°C for 30 min. The HUVEC cells were then seeded on collagen at $8.10^5$ cells per well. After monolayer formation (one day), a second layer of collagen 200 μL per well was prepared according to the same protocol and gently placed on top of the cell layer. The compounds of the invention were added to the cells at the adhesion step (concentration corresponding to the Inhibitory Concentration 50 ($IC_{50}$)). After 24 and 48 h of incubation at 37°C, tube formation was observed and pictures were taken.

***In vivo Studies***

Female (NOD-SCID, IL-2R$\gamma^{-/-}$) mice 5-6 weeks old were injected subcutaneously with MDA-MB-231 cells ($5.10^6$) at a single site on their right flanks. Tumors were allowed to grow to about 100 $mm^3$ before being pair-matched by tumor size into treatment groups (8 mice/group). One group was treated with vehicles (8 mice receiving cremofor 12.5%/ethanol 12.5%/ $H_2O$), one group was treated with 10 mg/kg of the compound **1** of the invention (8 mice receiving compound **1** + cremofor 12.5%/ethanol 12.5%/ $H_2O$), one group was treated with 50 mg/kg of the compound **1** of the invention (8 mice receiving compound **1**+ cremofor 12.5%/ethanol 12.5%/ $H_2O$). All groups received 3 times a week by oral gavages approximately 100 μL of a solution containing the compound **1** of the invention. Mice were weighed regularly to assess toxicity of the treatments and the tumors were measured with calipers (width x width x length x Pi/6) to determine tumor growth. Mice survival was assessed during the study and metastatic development was investigated in lung, spleen and liver.

***Inhibition of VEGF on NRP-1 or VEGF-R1 interaction***

Elisa test was performed covering a 96-well microplate (BD Biosciences, France, cat n° 353046) surface with 100 μL of 1X PBS, pH 7.4 with $Ca^{2+}$ and $Mg^{2+}$, (Lonza, France, cat n° 17-513F/12) solution containing 0.2 μg/mL of VEGF-R1 (R&D Systems, UK, cat n° 321-FL-050/CF) or 2 μg/mL of NRP-1 (R&D Systems, UK, cat n° 3870-N1-025). The plate was sealed and incubated overnight at 4°C. After 3 washes with 200 μL of 1X PBS 0.5% (v/v) Tween 20 (Sigma, France, cat n° 34078) (Buffer A), the plate was blocked by adding 200 μL of 1× PBS with 0.5% (w/v) BSA (Eurobio, France, cat n° GAUBSA01-65) and agitated at 37°C for 2 h. After washing five times with Buffer A, 50 μL/well of the compounds of the invention at 10 μM were added for the specific signal and 50 μL/well for the non-specific and max binding signal and incubated at 37°C for 1 hour, shacking. 50 μL of btVEGF (R&D Systems, UK, cat n° NFVEO) at 200 μg/mL for NRP-1 / 4 μg/mL Heparin (Sigma, USA, cat n°H3393-10KU)and 100 μg/mL for VEGF-R1 solution was added to each well; the plate was sealed and incubated at 37°C for 2 h, shacking. The last incubation was carried out after five washes with Buffer A, adding 100 μL/well Streptavidin-Horseradish Peroxidase (Amersham, Switzerland, cat n° RPN4401) diluted 1:8000 with buffer A. After 1 h shacking at 37°C under obscurity, the plate was washed 5 times

with buffer A and 100 µL of SuperSignal west picochemiluminescent substrate (Interchim, USA cat n° 34078) were added. Luminescence was quantified with an EnVision™ 2101 Multilabel reader (Perkin Elmer, USA) and the data analyzed by Wallac Envision manager software. Signal is given as relative light units (RLU). Non-specific binding is defined as the signal measured in the absence of receptor coated on the microplate. The specific binding was calculated as the difference between total binding and non-specific binding. The percentage of inhibition was calculated as following:

$$\text{Inhibition \%} = 100 \times \frac{\text{(Signal measured – non specific binding signal)}}{\text{(Max binding signal obtained without inhibitor - non specific binding signal without receptor)}}$$

***Measurement of binding affinity of compound 1 of the invention for NRP-1 and NRP-2 (in-vitro cell based assay)***

The effect of compound **1** of the invention on cell proliferation was tested according to the proliferation assay hereinabove described, in MDA-MB-231 cancer cells transfected with shRNA directed to NRP1 or NRP2 or with control shRNA.

***Statistical analysis***

Data reported here are the means ± SD of three/five independent experiments, each performed in triplicate. The statistical significance of results was evaluated by ANOVA test, with probability values **p*=0.05, ***p*=0.01, ****p*=0.001, being considered as significant.

**Results**

***Anti-angiogenic activity***

First, we tested the cytotoxic activity of compound **1** of the invention on endothelial cell lines (HUVEC cells) in culture.

As shown in the **Figure 1**, compound **1** of the invention inhibited both the adhesion (**Figure 1A**) and the proliferation (**Figure 1B**) of HUVEC cells in vitro.

It could therefore be hypothesized that compound **1** of the invention possesses anti-angiogenic activities. To confirm this hypothesis, in vitro experiments for assessing the formation of blood vessels were carried out. We measured the effect of compound **1** of the invention on tube formation (**Figures 2A** and **2B**).

As shown in **Figure 2**, compound **1** of the invention effectively inhibits the formation of new blood vessels, thus confirming the anti-angiogenic activity of compound **1** of the invention.

***Anti-tumor activity***

We then tested the anti-tumor activity of compound **1** of the invention. Experiments were carried out using MDA-MB-231 cell cultures. MDA-MB-231 is a breast cancer cell line.

**Figure 3** shows that compound **1** of the invention inhibits both the adhesion (**Figure 3A**) and the proliferation (**Figure 3B**) of MDA-MB-231 cells in vitro.

The cytotoxicity of compound **1** of the invention was confirmed on different cancer cell lines, using a wst-1 cytotoxic test. Results are presented on Table **1** below. Results showed that compound **1** of the invention is cytotoxic for all the tested cell lines, whatever the type of cancer (either solid cancer or haematological cancer) or their origin (breast, brain, etc...).

Table 1

| **Cell lines** | | **$IC_{50}$ µM Compound 1** |
|---|---|---|
| **Type** | **Name** | |
| **Solid cancer cells** | | |
| Breast | MDA-MB-231 | 0.7 ± 0.08 |
| | MDA-MB-468 | 0.5 ± 0.06 |

(continued)

| Cell lines | | IC$_{50}$ μM Compound 1 |
|---|---|---|
| **Type** | **Name** | |
| **Solid cancer cells** | | |
| Brain | U87 | 0.5 ± 0.02 |
| | SH-SY5Y | 0.1 ± 0.02 |
| Cervical | HeLa | 0.2 ± 0.06 |
| Colon | HCT-116 | 0.5 ± 0.07 |
| | HT-29 | 0.5 ± 0.01 |
| Fibrosarcoma | HT-1080 | 0.3 ± 0.01 |
| Lung | A549 | 0.4 ± 0.03 |
| | NCI-H460 | 0.5 ± 0.07 |
| Pancreas | Panc-10.5 | 0.5 ± 0.04 |
| **Hematological cancer cells** | | |
| Acute lymphoblastic leukemia | CEM | 0.1 ± 0.08 |
| Burkitt's lymphoma | DAUDI | 0.5 ± 0.02 |
| B lymphoma | VAL | 1 ± 0.06 |
| Mantle cell lymphoma | Granta-519 | 0.5 ± 0.07 |
| Chronic myeloid leukemia | ERY-1 | 0.2 ± 0.03 |

Furthermore, we analysed the effect of compound **1** of the invention on the motility of cancer cells, an essential step for invasion and metastasis. **Figure 4** shows that compound **1** of the invention has a significant anti-migratory effect on MDA-MB-231 cancer cells.

Then, the potential in vivo anti-cancer effect of compound **1** of the invention was tested on mice. NOD-SCID IL-2R$\gamma^{-/-}$ mice were injected with human breast cancer cells (MDA-MB-231). Once tumors grew up to 100 mm$^3$, mice were fed with compound **1** of the invention (10 or 50 mg/kg of body weight) or with vehicle (control), three times a week. Results of the experiment are shown in **Figure 5.**

Results show no significant change in body weight, demonstrating the absence of toxicity of the compound of the invention at both tested doses (**Figure 5A**).

Our results demonstrate that compound **1** of the invention inhibits the development of tumors. Indeed, it is clear on **Figure 5B** that the tumor size was smaller in treated mice than in the control.

***Inhibition pathway***

In order to characterize the effect of compound **1** of the invention, we then analyzed its effects on Neuropilin and VEGF pathways.

First, we carried out an in-vitro cell based assay for measuring the inhibition of proliferation of MDA-MB-231 cells treated with compound **1** of the invention, and with shRNA directed to NRP-1 or NRP-2. Measured IC$_{50}$ are presented in Table 2 below.

Table 2

| Clone | IC$_{50}$ (μM) |
|---|---|
| MDA-MB-231 | 0.7 ± 0.05 |
| MDA-MB-231 + control shRNA | 0.8 ± 0.05 |
| MDA-MB-231 + shRNA directed to NRP1 | 9 ± 0.08 |
| MDA-MB-231 + shRNA directed to NRP2 | 10 ± 0.05 |

As shown in Table 2, the effect of compound **1** of the invention is strongly impaired when cells are treated with a shRNA directed to NRP1 or NRP2. This result thus indicates that the effects of compound **1** of the invention are mediated by both receptors NRP1 and NRP2.

Then, we tested the effect of compound **1** (used at 10 μM) of the invention on the binding of VEGF-$A_{165b}$ on its receptors Neuropilin-1 (NRP-1) and VEGF-R1. Results are presented in the Table 3 below.

| | **Percent of binding inhibition (± SD)** |
|---|---|
| VEGF-$A_{165b}$ / NRP-1 | 32 ± 3.2 |
| VEGF-$A_{165b}$ / VEGF-R1 | 23 ± 2.2 |

## Claims

1. A compound of general formula (I),

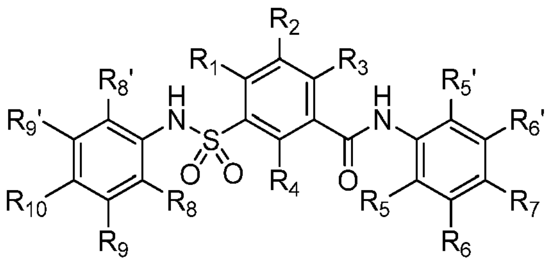

and salts or solvate thereof,
wherein
**$R^1$, $R^2$, $R^3$** and **$R^4$** are independently a hydrogen atom, or a group selected from alkyl, haloalkyl, alkenyl and haloalkenyl;
**$R^5$** and **$R^{5'}$** are independently a hydrogen atom, or a group selected from alkyl, haloalkyl, alkenyl, haloalkenyl, alkoxy, haloalkoxy, alkylamino and alkylthio, wherein at least one of **$R^5$** and **$R^{5'}$** is an alkoxy, haloalkoxy, alkylamino or alkylthio group;
**$R^6$, $R^{6'}$** and **$R^7$** are independently a hydrogen atom, or a group selected from alkyl, haloalkyl, alkenyl, haloalkenyl and alkoxy;
**$R^8$**, **$R^{8'}$**, **$R^9$** and **$R^{9'}$** are independently a hydrogen atom, or a group selected from alkyl, haloalkyl, alkenyl, haloalkenyl and alkoxy;
**$R^{10}$** is a group selected from alkyl, haloalkyl, alkenyl and haloalkenyl; for use in the treatment of a Neuropilin-related disease, disorder or condition in a subject in need thereof.

2. The compound for use according to claim **1**, being:

N-(2-ethoxyphenyl)-4-methyl-3-(N-(p-tolyl)sulfamoyl)benzamide

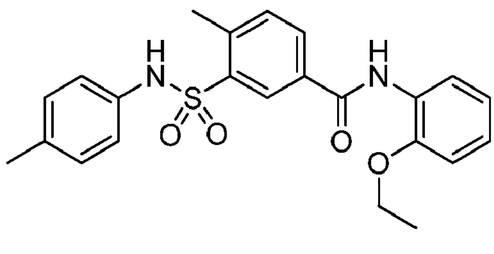

or a salt or solvate thereof.

3. A composition comprising a compound for use according to claim **1 or** claim **2**.

4. A pharmaceutical composition comprising a compound for use according to claim **1** or claim **2**, or salts, solvate or mixtures thereof, in association with at least one pharmaceutically acceptable vehicle.

5. A medicament comprising a compound for use according to claim **1** or claim **2**, or salts, solvate or mixtures thereof.

6. A method for manufacturing a compound according to claim **1** or claim **2**, where a compound of general formula (II),

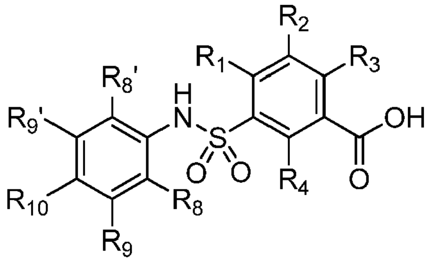

(II)

wherein $\mathbf{R^1}$, $\mathbf{R^2}$, $\mathbf{R^3}$, $\mathbf{R^4}$, $\mathbf{R^8}$, $\mathbf{R^{8'}}$, $\mathbf{R^9}$, $\mathbf{R^{9'}}$ and $\mathbf{R^{10}}$ are as defined in claim 1, is reacted through a peptide coupling reaction with an amine of general formula (III),

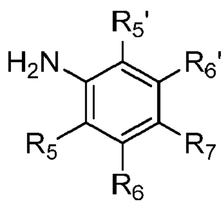

(III)

wherein $\mathbf{R^5}$, $\mathbf{R^{5'}}$, $\mathbf{R^6}$, $\mathbf{R^{6'}}$ and $\mathbf{R^7}$ are as described hereinabove,
to give compounds of general formula (I).

7. A compound for use according to claim **1** or **2**, a composition according to claim **3**, a pharmaceutical composition according to claim **4**, a medicament according to claim **5** or a compound manufactured by the method according to claim **6**, wherein the Neuropilin-related disease, disorder or condition is cancer.

8. A compound for use, a composition, a pharmaceutical composition or a medicament according to claim **7** for use for inhibiting the proliferation of cancer cells and/or for inhibiting the adhesion of cancer cells and/or for inhibiting the growth of a tumor and/or for inhibiting the number and the development of cancerous cells within the body of the subject and/or for inhibiting the occurrence of metastases.

9. A compound for use, a composition, a pharmaceutical composition or a medicament according to claim **7** or **8**, wherein the cancer is one of carcinoma, adenocarcinoma, lymphoma, blastoma, hepatoma, sarcoma; leukemia, such as, for example, squamous cell cancer; lung cancer, including small-cell lung cancer, non-small cell lung cancer, adenocarcinoma of the lung, and squamous carcinoma of the lung; cancer of the peritoneum; hepatocellular cancer; gastric or stomach cancer, including gastrointestinal cancer; pancreatic cancer, such as, for example, pancreatic carcinoma; fibrosarcoma, glioblastoma; neuroblastoma; cervical cancer; ovarian cancer; liver cancer; bladder cancer; hepatoma; breast cancer; colon cancer, such as, for example, colon adenocarcinoma; colorectal cancer, such as, for example, colorectal carcinoma; endometrial or uterine carcinoma; salivary gland carcinoma; osteosarcoma; kidney or renal cancer; liver cancer; prostate cancer, such as, for example, prostate adenocarcinoma; vulval cancer; thyroid cancer; hepatic carcinoma and various types of head and neck cancer; B-cell lymphoma, including low grade/follicular non-Hodgkin's lymphoma (NHL), small lymphocytic (SL) NHL, Burkitt's lymphoma, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, and Waldenstrom's Macroglobulinemia, chronic lymphocytic leukemia (CLL), acute lymphoblastic leukemia (ALL),

Hairy cell leukemia, chronic myeloblastic leukemia; and post-transplant lymphoproliferative disorder (PTLD), abnormal vascular proliferation associated with phakomatoses, edema, such as, for example that associated with brain tumors; and Meigs' syndrome.

**10.** A compound for use, a composition, a pharmaceutical composition or a medicament according to anyone of claims **7** to **9**, wherein said subject is diagnosed with a cancer and/or underwent a surgical operation to remove a tumor and/or was previously treated with an anti-cancer treatment, and/or is simultaneously treated with an anti-cancer treatment, and/or is planned to be treated with an anti-cancer treatment, wherein the anti-cancer treatment may be chemotherapy and/or radiotherapy.

**11.** A compound for use according to claim **1** or **2**, a composition according to claim **3**, a pharmaceutical composition according to claim **4**, a medicament according to claim **5** or a compound manufactured by the method according to claim **6**, wherein the Neuropilin-related disease, disorder or condition is an angiogenic disease, disorder or condition.

**12.** A compound for use, a composition, a pharmaceutical composition or a medicament according to claim **11** for use for inhibiting the formation of neovessels and/or the adhesion of endothelial cells and/or the growth, proliferation, development or division of endothelial cells.

**13.** A compound for use, a composition, a pharmaceutical composition or a medicament according to claim **11** or claim **12**, wherein the angiogenic disease, disorder or condition is an ophthalmic disease, a disease of the renal and/or urogenital tract; a disease of the digestive tract; a disease of the respiratory tract; a disease of bones or joints; a skin disease; an autoimmune disease; is consecutive to a graft; is a post-surgery reaction, a hyperproliferation or a hypertrophy; abnormal wound healing; chronic inflammation; a disease of the cardiovascular system or of blood or an infection.

A

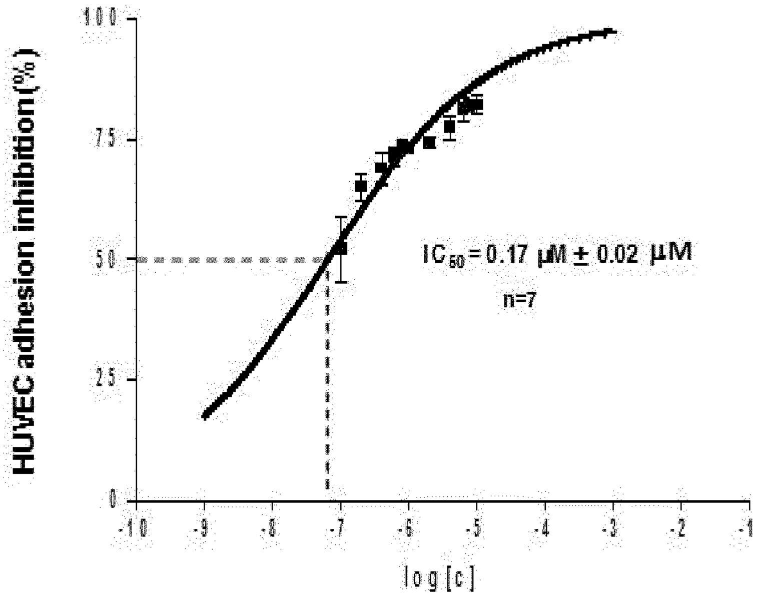
HUVEC adhesion inhibition (%)
100
75
50
25
0
-10
-9
-8
-7
-6
-5
-4
-3
-2
-1
log [c]
IC50 = 0.17 µM ± 0.02 µM
n=7

B

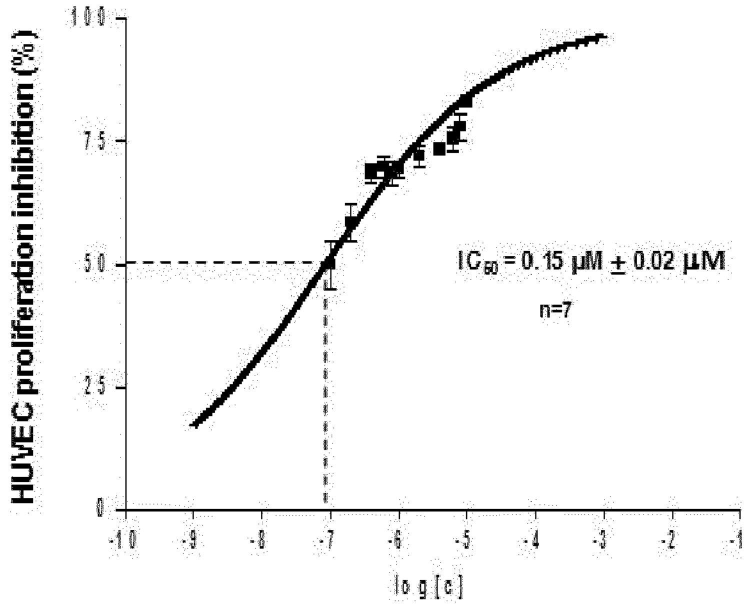
HUVEC proliferation inhibition (%)
100
75
50
25
0
-10
-9
-8
-7
-6
-5
-4
-3
-2
-1
log [c]
IC50 = 0.15 µM ± 0.02 µM
n=7

FIG. 1

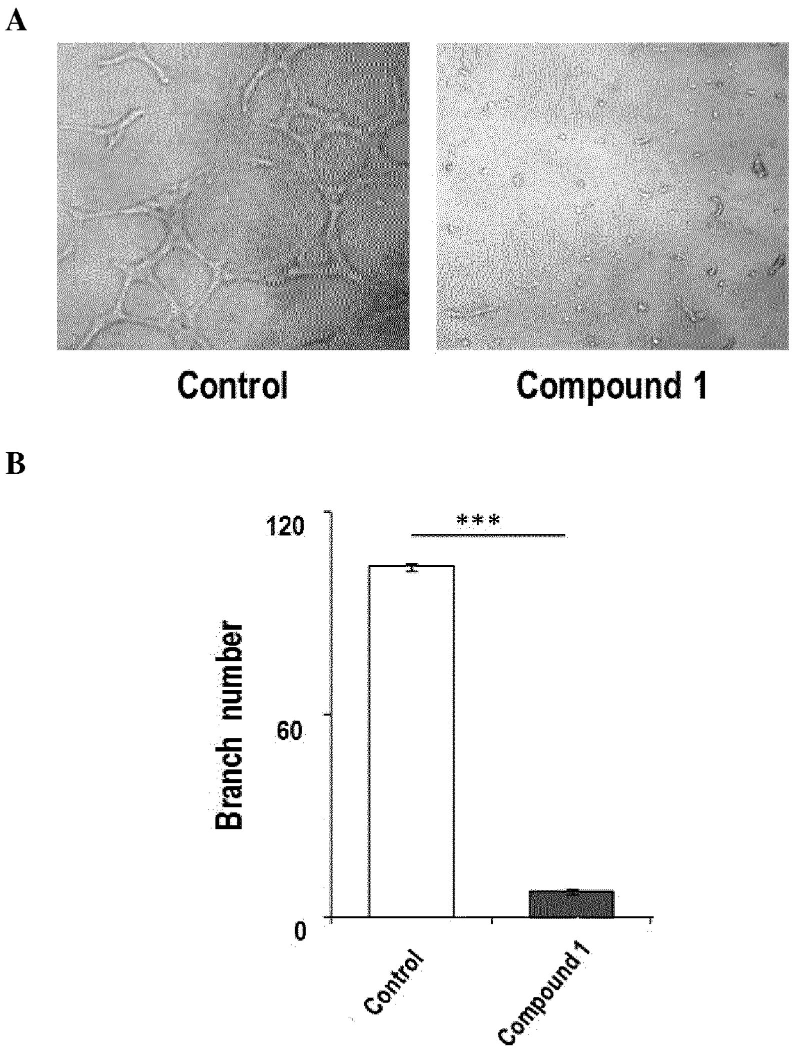
A
Control
Compound 1
B
120
60
0
Branch number
***
Control
Compound 1

FIG. 2

A

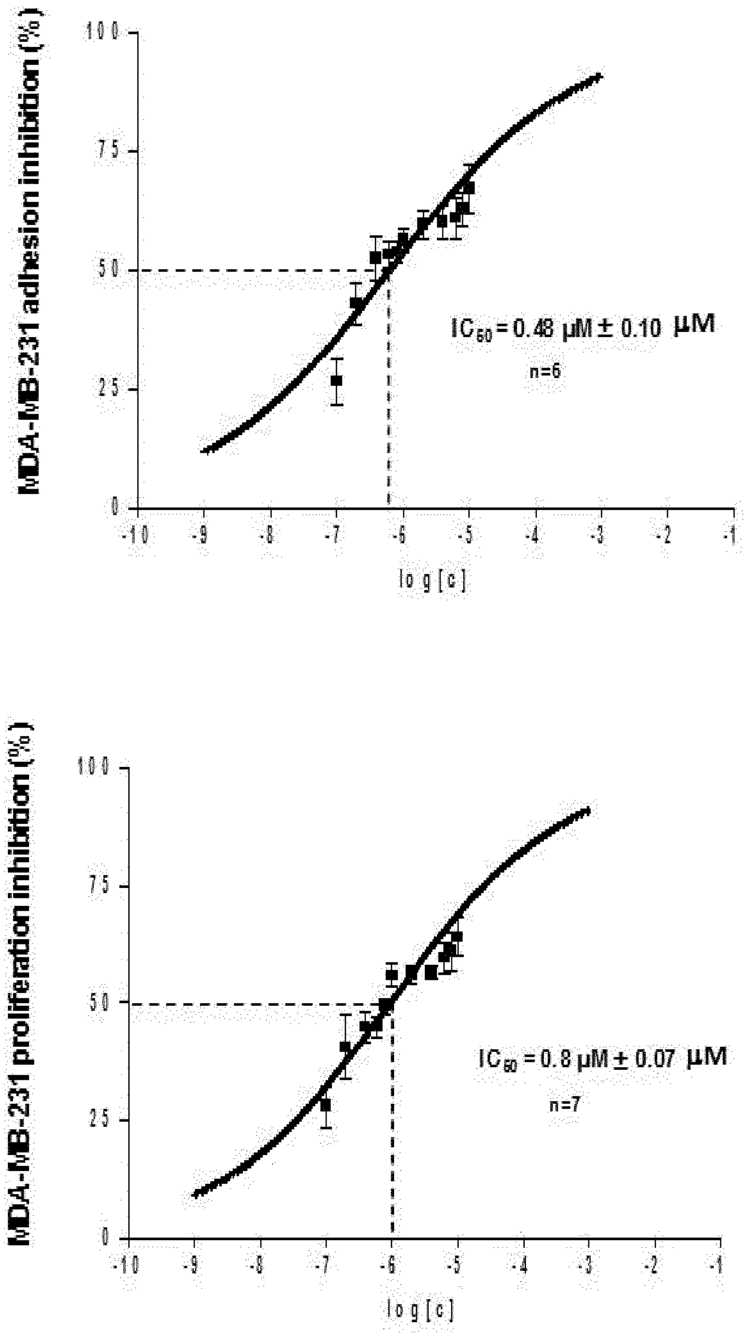
MDA-MB-231 adhesion inhibition (%)
100
75
50
25
0
-10
-9
-8
-7
-6
-5
-4
-3
-2
-1
log[c]
$IC_{50}$ = 0.48 µM ± 0.10 µM
n=6
B
MDA-MB-231 proliferation inhibition (%)
100
75
50
25
0
-10
-9
-8
-7
-6
-5
-4
-3
-2
-1
log[c]
$IC_{50}$ = 0.8 µM ± 0.07 µM
n=7

FIG. 3

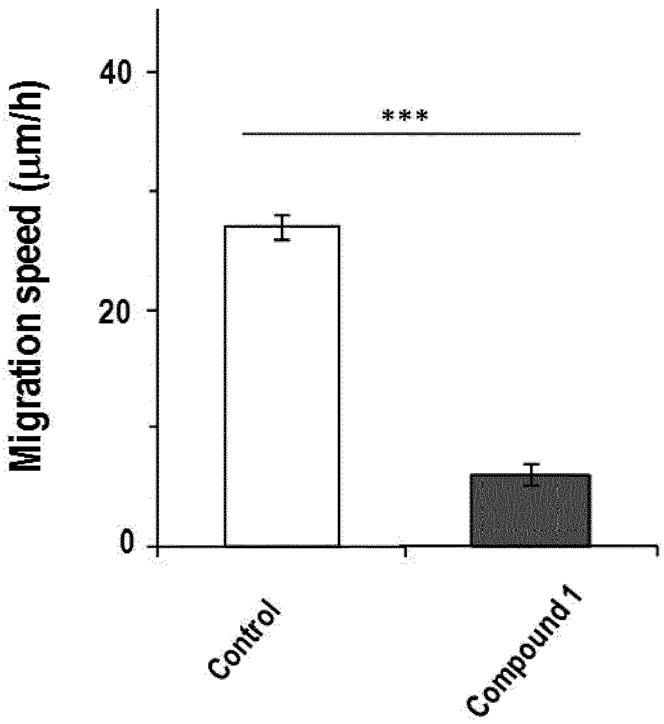


FIG. 4

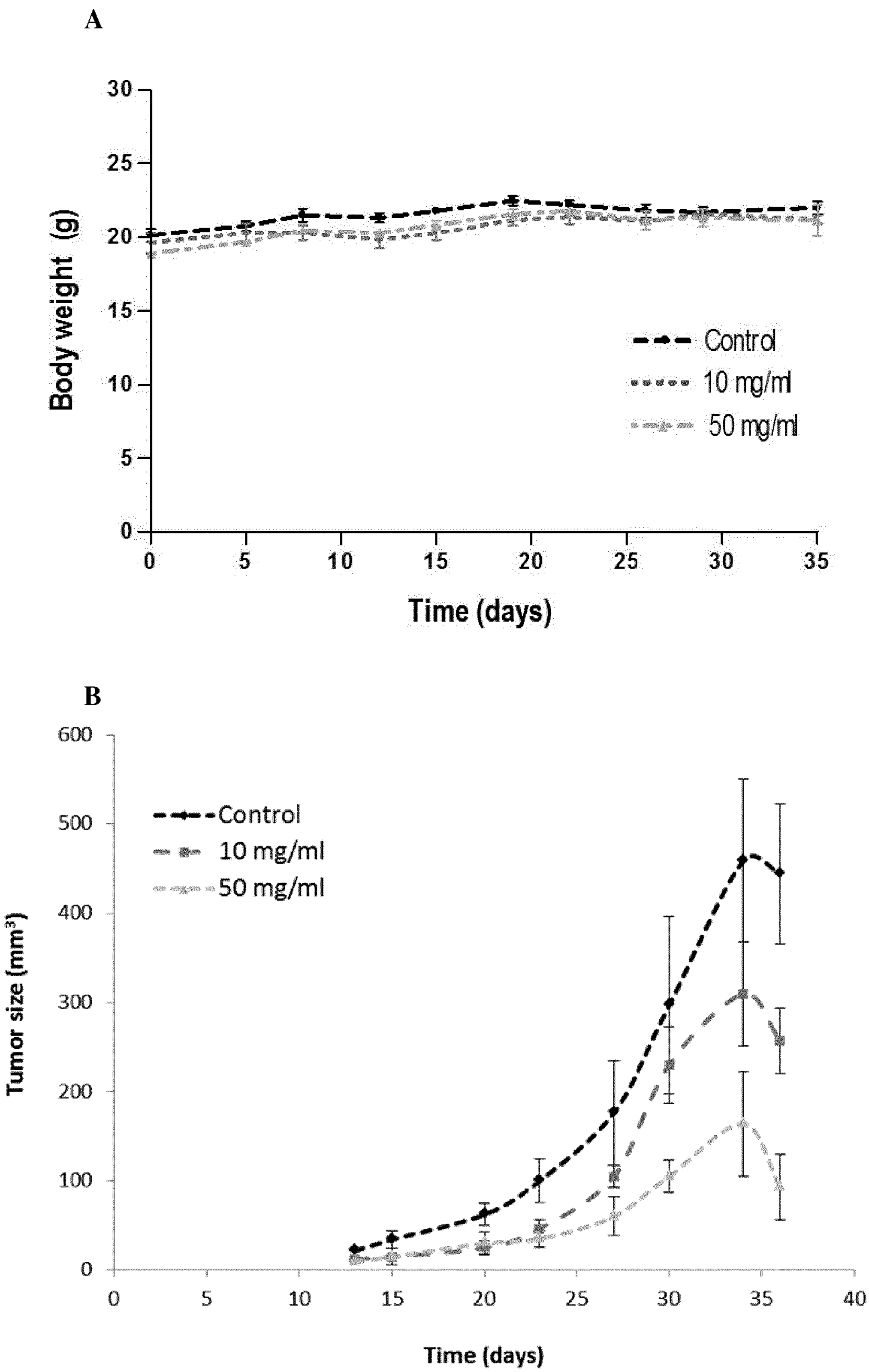
A
30
25
20
15
10
5
0
Body weight (g)
0
5
10
15
20
25
30
35
Time (days)
Control
10 mg/ml
50 mg/ml
B
600
500
400
300
200
100
0
Tumor size (mm³)
0
5
10
15
20
25
30
35
40
Time (days)
Control
10 mg/ml
50 mg/ml

FIG. 5

Europäisches Patentamt
European Patent Office
Office européen des brevets

# EUROPEAN SEARCH REPORT

Application Number
EP 13 17 5813

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | JARVIS ET AL.: "Small molecule inhibitors of the neuropilin-1 vascular endothelial growth factor A (VEGF-A) interaction", J. MED. CHEM., vol. 53, 2 December 2010 (2010-12-02), pages 2215-26, XP002719561, USA ISSN: 0022-2623, DOI: 10.1021/jm901755g * the whole document * ----- | 1-13 | INV. A61K31/18 A61P35/00 A61P9/10 |
| X | WO 2008/022171 A1 (BOEHRINGER INGELHEIM INT [DE]; BOEHRINGER INGELHEIM PHARMA [DE]; INGRA) 21 February 2008 (2008-02-21) * page 9; compound 3 * * page 11; compounds 2,5 * * page 12; compound 6 * * page 14; compound 5 * * claims * ----- | 1,3-6, 11-13 | |
| A | US 2010/087415 A1 (WHITTEN JEFFREY P [US] ET AL) 8 April 2010 (2010-04-08) * the whole document * ----- | 1-13 | TECHNICAL FIELDS SEARCHED (IPC) |
| A | WO 2011/088561 A1 (UNIV MANITOBA [CA]; YAO XIAOJIAN [CA]; AO ZHUJUN [CA]) 28 July 2011 (2011-07-28) * formula (I) * * claims * ----- -/-- | 1-13 | A61K |

The present search report has been drawn up for all claims

2

| Place of search | Date of completion of the search | Examiner |
|---|---|---|
| Munich | 11 February 2014 | Villa Riva, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 5813

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KHURANA ROHIT ET AL: "Role of angiogenesis in cardiovascular disease - A critical appraisal", CIRCULATION, AMERICAN HEART ASSOCIATION, INC, US, vol. 112, no. 12, 20 September 2005 (2005-09-20), pages 1813-1824, XP009170535, ISSN: 0009-7322, DOI: 10.1161/CIRCULATIONHA.105.535294 * tables * ----- | 1-13 | |

TECHNICAL FIELDS SEARCHED (IPC)

The present search report has been drawn up for all claims

2

| Place of search | Date of completion of the search | Examiner |
|---|---|---|
| Munich | 11 February 2014 | Villa Riva, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 17 5813

This annex lists the patent family members relating to the patent documents cited in the above-mentioned European search report. The members are as contained in the European Patent Office EDP file on The European Patent Office is in no way liable for these particulars which are merely given for the purpose of information.

11-02-2014

| Patent document cited in search report | Publication date | Patent family member(s) | Publication date |
|---|---|---|---|
| WO 2008022171 A1 | 21-02-2008 | US 2010016310 A1<br>WO 2008022171 A1 | 21-01-2010<br>21-02-2008 |
| US 2010087415 A1 | 08-04-2010 | US 2010087415 A1<br>US 2012149673 A1 | 08-04-2010<br>14-06-2012 |
| WO 2011088561 A1 | 28-07-2011 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03035100 A
- US 7335357 B
- WO 2009099959 A
- WO 9955855 A
- WO 2011143408 A


### Non-patent literature cited in the description


- **ELLIS LM.** *Mol Cancer Ther,* 2006, vol. 5 (5), 1099-107
- **JARVIS et al.** *J. Med. Chem.,* 2010, vol. 53, 2215-26